# EUROPEAN PATENT SPECIFICATION

(11) **EP 0 883 600 B1**
(45) Date of publication and mention of the grant of the patent: **24.10.2001**
(21) Application number: 97904114.2
(22) Date of filing: 05.02.1997
(51) Int. Cl.: C07C 219/06, C11D 1/62

(54) **CATIONIC DETERGENT COMPOUNDS**
KATIONISCHE WASCH-UND REINIGUNGSMITTEL
COMPOSES CATIONIQUES DETERGENTS

(30) Priority: 27.02.1996 GB 9604096
(43) Date of publication of application: 16.12.1998
(73) Proprietor: THE PROCTER & GAMBLE COMPANY, Cincinnati, Ohio 45202 (US)
(72) Inventor: HEINZMAN, Stephen Wayne, Gosforth, Newcastle upon Tyne NE3 2JP (GB)
(74) Representative: Peet, Jillian Wendy
(86) International application number: US9701529
(87) International publication number: WO9731889

(56) References cited:
- EP-A- 0 753 565
- WO-A-95/20640
- WO-A-95/24460
- WO-A-96/03370
- DE-A- 3 402 146
- US-A- 2 189 397
- US-A- 3 781 204
- US-A- 3 956 479
- ACTA CHEMICAL SCANDINAVICA, vol. 16, 1962, COPENHAGEN DK, pages 1927-1935, XP002031159 B. HANSEN: "Kinetics of the alkaline hydrolysis of aminoalkyl esters of carboxylic acids"

## Description

The present invention relates to selected cationic ester surfactants which are suitable for use in laundry and dish washing methods.

### Background to the invention

The satisfactory removal of greasy soils/stains, that is soils/stains having a high proportion of triglycerides or fatty acids, is a challenge faced by the formulator of detergent compositions for use in machine laundry and dishwashing methods. Surfactant components have traditionally been employed in detergent products to facilitate the removal of such greasy soils/stains.

In particular, surfactant systems comprising cationic ester surfactants have been described for use in greasy soil/stain removal. By cationic ester surfactants it is meant those compounds having surfactant properties which comprise at least one ester (i.e. -COO-) linkage and at least one cationically charged group. The cationically charged group is often an ammonium or substituted ammonium group.

For example, EP-B-21,491 discloses detergent compositions containing a nonionic/cationic surfactant mixture and a builder mixture comprising aluminosilicate and polycarboxylate builder. The cationic surfactant may be a cationic ester. Improved particulate and greasy/oily soil removal is described.

US-A-4,228,042 discloses biodegradable cationic surfactants, including cationic ester surfactants for use in detergent compositions to provide greasy/oily soil removal. The combination of these cationic surfactants with nonionic surfactants in compositions designed for particulate soil removal is also described. Anionic surfactants are disclosed as optional components of the compositions, but are present at low levels relative to the cationic surfactant component.

US-A-4,239,660 discloses laundry detergent compositions containing cationic ester surfactant and nonionic surfactant at defined weight ratios and an alkalinity source. The alkalinity source enables a wash solution having a pH of from 8 to 10 to be formed within 3 minutes of dissolution of the composition in water at 100°F (37°C) at a solution concentration of 0.15%.

US-A-4,260,529 discloses laundry detergent compositions having a pH of no greater than 11 containing cationic ester surfactant and nonionic surfactant at defined weight ratios. Anionic surfactants are disclosed as optional components of the compositions, but are present at low levels relative to the cationic ester surfactant component.

The Applicants have now found that a problem with the use of certain cationic ester surfactants is the tendency for the ester linkage to hydrolytically cleave, thereby breaking up the surfactant molecule, under the wash conditions of a typical laundry or dishwashing method. Surfactant performance in the wash is thereby compromised.

It has been established that enhanced stability to such hydrolytic cleavage, can be provided if the ester linkage is separated from the cationically charged group by a spacer group consisting of a chain comprising at least three atoms selected from carbon, nitrogen and oxygen atoms. Thus, the problem of break up of the surfactant surfactant molecule, under the wash conditions of a typical laundry or dishwashing method, is ameliorated, and the surfactant cleaning performance enhanced.

The prior art documents cited above include a general description of cationic ester surfactants in which the ester linkage may be separated from the cationically charged group by an alkyl chain comprising from one to five carbon atoms. Choline ester surfactants having alkyl chains comprising two carbon atoms are however, stated to be preferred and are exemplified in detail. None of the documents provides any teaching of the enhanced stablity to hydrolysis possessed by the present cationic ester surfactants in which the chain comprises at least three atoms.

### Summary of the Invention

According to the present invention there is provided a cationic ester surfactant.

The cationic ester surfactant is selected from those having the formula: 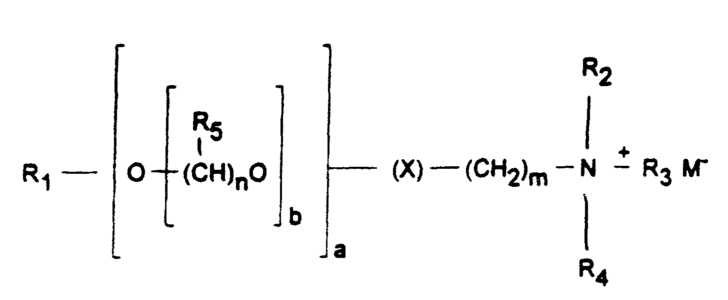 wherein R₁ is a C₅-C₃₁ linear or branched alkyl, alkenyl or alkaryl chain; X is selected from the group consisting of COO, OCO, OCOO, OCONH and NHCOO; R₂, R₃, and R₄ are independently selected from the group consisting of CH₃ and CH₂CH₂OH and R₅ is independently H or a C₁-C₃ alkyl group; wherein the value of n lies in the range of from 0 to 8, the value of b lies in the range from 0 to 20, the value of a is either 0 or 1, and the value of m is from 3 to 8.

### Detailed description of the invention

### Cationic ester surfactant

The surfactant of the present invention is a cationic ester surfactant, that is a compound having surfactant properties comprising at least one ester (ie -COO-) linkage and at least one cationically charged group. The cationically charged group is preferably an ammonium or substituted ammonium group.

The cationic ester surfactants are those having the formula: 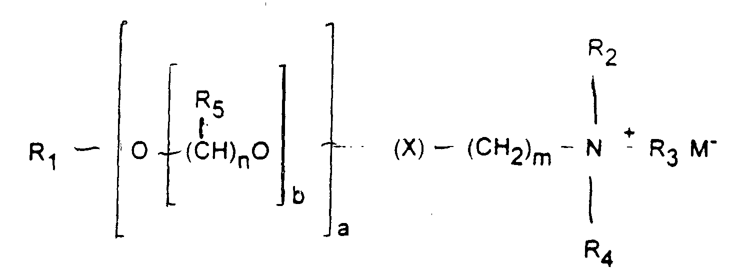 wherein R₁ is a C₅-C₃₁ linear or branched alkyl, alkenyl or alkaryl chain; X is selected from the group consisting of COO, OCO, OCOO, OCONH and NHCOO; R₂, R₃, and R₄ are independently selected from the group consisting of CH₃ and CH₂CH₂OH and R₅ is independently H or a C₁-C₃ alkyl group; wherein the value of n lies in the range of from 0 to 8, the value of b lies in the range from 0 to 20, the value of a is either 0 or 1, and the value of m is from 3 to 8.

M is selected from the group consisting of halide, methyl sulfate, sulfate, and nitrate, more preferably methyl sulfate, chloride, bromide or iodide.

Preferred water dispersible cationic ester surfactants are the 'propyl choline' esters having the formula: 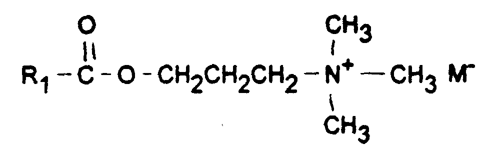 wherein R₁ is a C₁₁-C₁₉ linear or branched alkyl chain.

Particularly preferred choline esters of this type include the stearoyl propyl choline ester quaternary methylammonium halides (R¹=C₁₇ alkyl), palmitoyl propyl choline ester quaternary methylammonium halides (R¹=C₁₅ alkyl), myristoyl propyl choline ester quaternary methylammonium halides (R¹=C₁₃ alkyl), lauroyl propyl choline ester methylammonium halides (R¹=C₁₁ alkyl), cocoyl propyl choline ester quaternary methylammonium halides (R¹=C₁₁₋C₁₃ alkyl), tallowyl propyl choline ester quaternary methylammonium halides (R¹=C₁₅₋C₁₇ alkyl), and any mixtures thereof.

The particularly preferred cationic esters, given above, may be synthesized by the direct esterification of a fatty acid (R₁COOH), fatty acid halide (R₁COOHal) or fatty acid ester (R₁ COORₙ) of the desired chain length with dimethylaminopropanol, in the presence of an acid catalyst, such as methanesulfonic or sulfuric acid. The reaction product is then quaternized with a 'quaternizing agent' such as methyl halide, methyltosylate or dimethylsulfate forming the desired cationic ester.

Alternatively, they may be synthesized by the direct esterification of a fatty acid (R₁COOH), fatty acid halide (R₁COOHal) or fatty acid ester (R₁COORₙ) of the desired chain length together with 2-halopropanol, in the presence of an acid catalyst, such as methanesulfonic or sulfuric acid. The reaction product is then quaternized with trimethylamine, forming the desired cationic ester.

The quaternization steps in the above synthetic routes are preferably carried out in the presence of a solvent such as ethanol toluene, propylene glycol or preferably a fatty alcohol ethoxylate such as C₁₀-C₁₈ fatty alcohol ethoxylate having a degree of ethoxylation of from 3 to 50 ethoxy groups per mole. It can be useful to have an anionic surfactant, such as linear alkyl benzene sulfonate present during the quaternization step to disrupt the formation of any aqueous gel phases.

Analogous synthetic routes may be employed to make other cationic esters in accord with the invention.

Thus, a route involving esterification of an, optionally substituted, aminoalkanol with a fatty acid, fatty acid ester or fatty acid halide to form an aminoalkanol ester followed by quaternization of the amino group with an, optionally substituted, alkyl halide to obtain the cationic ester product is generally envisaged. Alternatively, a route involving esterification of an, optionally substituted, 2-haloalkanol with a fatty acid or fatty acid halide to form a 2-haloalkanol ester followed by reaction with an, optionally substituted, trialkylamine, to form the cationic ester is also generally envisaged.

### Surfactant systems

Surfactant systems herein comprise the cationic ester surfactant in accord with the present invention in combination with an additional surfactant selected from nonionic, non-ester cationic, ampholytic, amphoteric and zwitterionic surfactants and mixtures thereof.

A typical listing of anionic, nonionic, ampholytic, and zwitterionic classes, and species of these surfactants, is given in U.S.P. 3,929,678 issued to Laughlin and Heuring on December 30, 1975. Further examples are given in "Surface Active Agents and Detergents" (Vol. I and II by Schwartz, Perry and Berch). A list of suitable cationic surfactants is given in U.S.P. 4,259,217 issued to Murphy on March 31, 1981.

Surfactant sytems comprising anionic and/or nonionic surfactants in combination with the cationic ester surfactants of the invention are preferred herein. Most preferably the surfactant systems comprise both anionic and nonionic surfactants in combination with the cationic ester surfactants of the invention.

The weight ratio of anionic surfactant to cationic ester surfactant in the surfactant system is preferably from 3:1 to 50:1, more preferably from 4:1 to 40:1, most preferably from 5:1 to 20:1.

The weight ratio of nonionic surfactant to cationic ester surfactant in the surfactant system is preferably from 3:1 to 50:1, more preferably from 4:1 to 40:1, most preferably from 5:1 to 20:1.

### Anionic surfactant

Essentially any anionic surfactants useful for detersive purposes are suitable. These can include salts (including, for example, sodium, potassium, ammonium, and substituted ammonium salts such as mono-, di- and triethanolamine salts) of the anionic sulfate, sulfonate, carboxylate and sarcosinate surfactants. Anionic sulfate surfactants are preferred.

Other anionic surfactants include the isethionates such as the acyl isethionates, N-acyl taurates, fatty acid amides of methyl tauride, alkyl succinates and sulfosuccinates, monoesters of sulfosuccinate (especially saturated and unsaturated C₁₂-C₁₈ monoesters) diesters of sulfosuccinate (especially saturated and unsaturated C₆-C₁₄ diesters), N-acyl sarcosinates. Resin acids and hydrogenated resin acids are also suitable, such as rosin, hydrogenated rosin, and resin acids and hydrogenated resin acids present in or derived from tallow oil.

### Anionic sulfate surfactant

Anionic sulfate surfactants suitable for use herein include the linear and branched primary and secondary alkyl sulfates, alkyl ethoxysulfates, fatty oleoyl glycerol sulfates, alkyl phenol ethylene oxide ether sulfates, the C₅-C₁₇ acyl-N-(C₁-C₄ alkyl) and -N-(C₁-C₂ hydroxyalkyl) glucamine sulfates, and sulfates of alkylpolysaccharides such as the sulfates of alkylpolyglucoside (the nonionic nonsulfated compounds being described herein).

Alkyl sulfate surfactants are preferably selected from the linear and branched primary C₁₀-C₁₈ alkyl sulfates, more preferably the C₁₁-C₁₅ branched chain alkyl sulfates and the C₁₂-C₁₄ linear chain alkyl sulfates.

Alkyl ethoxysulfate surfactants are preferably selected from the group consisting of the C₁₀-C₁₈ alkyl sulfates which have been ethoxylated with from 0.5 to 20 moles of ethylene oxide per molecule. More preferably, the alkyl ethoxysulfate surfactant is a C₁₁-C₁₈, most preferably C₁₁-C₁₅ alkyl sulfate which has been ethoxylated with from 0.5 to 7, preferably from 1 to 5, moles of ethylene oxide per molecule.

A particularly preferred aspect of the invention employs mixtures of the preferred alkyl sulfate and alkyl ethoxysulfate surfactants. Such mixtures have been disclosed in PCT Patent Application No. WO 93/18124.

### Anionic sulfonate surfactant

Anionic sulfonate surfactants suitable for use herein include the salts of C₅-C₂₀ linear alkylbenzene sulfonates, alkyl ester sulfonates, C₆-C₂₂ primary or secondary alkane sulfonates, C₆-C₂₄ olefin sulfonates, sulfonated polycarboxylic acids, alkyl glycerol sulfonates, fatty acyl glycerol sulfonates, fatty oleyl glycerol sulfonates, and any mixtures thereof.

### Anionic carboxylate surfactant

Suitable anionic carboxylate surfactants include the alkyl ethoxy carboxylates, the alkyl polyethoxy polycarboxylate surfactants and the soaps ('alkyl carboxyls'), especially certain secondary soaps as described herein.

Suitable alkyl ethoxy carboxylates include those with the formula RO(CH₂CH₂O)ₓ CH₂COO⁻M⁺ wherein R is a C₆ to C₁₈ alkyl group, x ranges from O to 10, and the ethoxylate distribution is such that, on a weight basis, the amount of material where x is 0 is less than 20 % and M is a cation. Suitable alkyl polyethoxy polycarboxylate surfactants include those having the formula RO-(CHR₁-CHR₂-O)-R₃ wherein R is a C₆ to C₁₈ alkyl group, x is from 1 to 25, R₁ and R₂ are selected from the group consisting of hydrogen, methyl acid radical, succinic acid radical, hydroxysuccinic acid radical, and mixtures thereof, and R₃ is selected from the group consisting of hydrogen, substituted or unsubstituted hydrocarbon having between 1 and 8 carbon atoms, and mixtures thereof.

Suitable soap surfactants include the secondary soap surfactants which contain a carboxyl unit connected to a secondary carbon. Preferred secondary soap surfactants for use herein are water-soluble members selected from the group consisting of the water-soluble salts of 2-methyl-1-undecanoic acid, 2-ethyl-1-decanoic acid, 2-propyl-1-nonanoic acid, 2-butyl-1-octanoic acid and 2-pentyl-1-heptanoic acid. Certain soaps may also be included as suds suppressors.

### Alkali metal sarcosinate surfactant

Other suitable anionic surfactants are the alkali metal sarcosinates of formula R-CON (R¹) CH₂ COOM, wherein R is a C₅-C₁₇ linear or branched alkyl or alkenyl group, R¹ is a C₁-C₄ alkyl group and M is an alkali metal ion. Preferred examples are the myristyl and oleoyl methyl sarcosinates in the form of their sodium salts.

### Alkoxylated nonionic surfactant

Essentially any alkoxylated nonionic surfactants are suitable herein. The ethoxylated and propoxylated nonionic surfactants are preferred.

Preferred alkoxylated surfactants can be selected from the classes of the nonionic condensates of alkyl phenols, nonionic ethoxylated alcohols, nonionic ethoxylated/propoxylated fatty alcohols, nonionic ethoxylate/propoxylate condensates with propylene glycol, and the nonionic ethoxylate condensation products with propylene oxide/ethylene diamine adducts.

### Nonionic alkoxylated alcohol surfactant

The condensation products of aliphatic alcohols with from 1 to 25 moles of alkylene oxide, particularly ethylene oxide and/or propylene oxide, are suitable for use herein. The alkyl chain of the aliphatic alcohol can either be straight or branched, primary or secondary, and generally contains from 6 to 22 carbon atoms. Particularly preferred are the condensation products of alcohols having an alkyl group containing from 8 to 20 carbon atoms with from 2 to 10 moles of ethylene oxide per mole of alcohol.

### Nonionic polyhydroxy fatty acid amide surfactant

Polyhydroxy fatty acid amides suitable for use herein are those having the structural formula R²CONR¹Z wherein : R1 is H, C₁-C₄ hydrocarbyl, 2-hydroxy ethyl, 2-hydroxy propyl, ethoxy, propoxy, or a mixture thereof, preferable C1-C4 alkyl, more preferably C₁ or C₂ alkyl, most preferably C₁ alkyl (i.e., methyl); and R₂ is a C₅-C₃₁ hydrocarbyl, preferably straight-chain C₅-C₁₉ alkyl or alkenyl, more preferably straight-chain C₉-C₁₇ alkyl or alkenyl, most preferably straight-chain C₁₁-C₁₇ alkyl or alkenyl, or mixture thereof; and Z is a polyhydroxyhydrocarbyl having a linear hydrocarbyl chain with at least 3 hydroxyls directly connected to the chain, or an alkoxylated derivative (preferably ethoxylated or propoxylated) thereof. Z preferably will be derived from a reducing sugar in a reductive amination reaction; more preferably Z is a glycityl.

### Nonionic fatty acid amide surfactant

Suitable fatty acid amide surfactants include those having the formula: R⁶CON(R⁷)₂ wherein R⁶ is an alkyl group containing from 7 to 21, preferably from 9 to 17 carbon atoms and each R⁷ is selected from the group consisting of hydrogen, C₁-C₄ alkyl, C₁-C₄ hydroxyalkyl, and-(C₂H₄O)ₓH, where x is in the range of from 1 to 3.

### Nonionic alkylpolysaccharide surfactant

Suitable alkylpolysaccharides for use herein are disclosed in U.S. Patent 4,565,647, Llenado, issued January 21, 1986, having a hydrophobic group containing from 6 to 30 carbon atoms and a polysaccharide, e.g., a polyglycoside, hydrophilic group containing from 1.3 to 10 saccharide units.

Preferred alkylpolyglycosides have the formula

R²O(CₙH₂ₙO)t(glycosyl)ₓ

wherein R² is selected from the group consisting of alkyl, alkylphenyl, hydroxyalkyl, hydroxyalkylphenyl, and mixtures thereof in which the alkyl groups contain from 10 to 18 carbon atoms; n is 2 or 3; t is from 0 to 10, and x is from 1.3 to 8. The glycosyl is preferably derived from glucose.

### Amphoteric surfactant

Suitable amphoteric surfactants for use herein include the amine oxide surfactants and the alkyl amphocarboxylic acids.

Suitable amine oxides include those compounds having the formula R³(OR⁴)ₓN⁰(R⁵)₂ wherein R³ is selected from an alkyl, hydroxyalkyl, acylamidopropoyl and alkyl phenyl group, or mixtures thereof, containing from 8 to 26 carbon atoms; R⁴ is an alkylene or hydroxyalkylene group containing from 2 to 3 carbon atoms, or mixtures thereof; x is from 0 to 5, preferably from 0 to 3; and each R⁵ is an alkyl or hydroxyalkyl group containing from 1 to 3, or a polyethylene oxide group containing from 1 to 3 ethylene oxide groups. Preferred are C₁₀-C₁₈ alkyl dimethylamine oxide, and C₁₀₋₁₈ acylamido alkyl dimethylamine oxide.

A suitable example of an alkyl aphodicarboxylic acid is Miranol(TM) C2M Conc. manufactured by Miranol, Inc., Dayton, NJ.

### Zwitterionic surfactant

Zwitterionic surfactants can also be incorporated into the detergent compositions hereof. These surfactants can be broadly described as derivatives of secondary and tertiary amines, derivatives of heterocyclic secondary and tertiary amines, or derivatives of quaternary ammonium, quaternary phosphonium or tertiary sulfonium compounds. Betaine and sultaine surfactants are exemplary zwitterionic surfactants for use herein.

Suitable betaines are those compounds having the formula R(R')₂N⁺R²COO- wherein R is a C₆-C₁₈ hydrocarbyl group, each R¹ is typically C₁-C₃ alkyl, and R² is a C₁-C₅ hydrocarbyl group. Preferred betaines are C₁₂₋₁₈ dimethyl-ammonio hexanoate and the C₁₀₋₁₈ acylamidopropane (or ethane) dimethyl (or diethyl) betaines. Complex betaine surfactants are also suitable for use herein.

### Cationic surfactants

Additional cationic surfactants can also be used in the detergent compositions herein. Suitable cationic surfactants include the quaternary ammonium surfactants selected from mono C₆-C₁₆, preferably C₆-C₁₀ N-alkyl or alkenyl ammonium surfactants wherein the remaining N positions are substituted by methyl, hydroxyethyl or hydroxypropyl groups.

### Detergent compositions

Detergent compositions may be formulated containing the cationic esters of the present invention, and the above mentioned cationic ester containing surfactant systems, in combination with additional detergent components.

Preferably, the level of incorporation of the surfactant system is from 1% to 95%, more preferably from 3% to 60%, most preferably from 5% to 40% by weight of the detergent composition. The level of incorporation of the cationic ester surfactant is preferably from 0.1% to 50%, more preferably from 0.5% to 30%, most preferably from 1.0% to 10% by weight of the detergent composition.

Detergent compositions containing one or more additional detergent components selected from the group consisting of an alkalinity system, bleaches, builders, organic polymeric compounds, enzymes, suds suppressors, lime soap dispersants, soil suspension and anti-redeposition agents and corrosion inhibitors are thus envisaged. The precise nature of these additional components, and levels of incorporation thereof will depend on the physical form of the composition, and the precise nature of the washing operation for which it is to be used.

### Alkalinity system

Detergent compositions herein may contain from 1.5% to 95%, preferably from 5% to 60%, most preferably from 10% to 40% by weight of the composition of an alkalinity system comprising components capable of providing alkalinity species in solution. By alkalinity species it is meant herein: carbonate, bicarbonate, hydroxide and the various silicate anions. Such alkalinity species can be formed for example, when alkaline salts selected from alkali metal or alkaline earth carbonate, bicarbonate, hydroxide or silicate, including crystalline layered silicate, salts and any mixtures thereof are dissolved in water. Alkali metal percarbonate and persilicate salts are also suitable sources of alkalinity species.

Examples of carbonates are the alkaline earth and alkali metal carbonates, including sodium carbonate and sesqui-carbonate and any mixtures thereof with ultra-fine calcium carbonate such as are disclosed in German Patent Application No. 2,321,001 published on November 15, 1973. Alkali metal percarbonate salts are also suitable sources of carbonate species and are described in more detail in the section 'inorganic perhydrate salts' herein.

### Suitable silicates include the water soluble sodium silicates with an SiO₂:

Na₂O ratio of from 1.0 to 2.8, with ratios of from 1.6 to 2.0 being preferred, and 2.0 ratio being most preferred. The silicates may be in the form of either the anhydrous salt or a hydrated salt. Sodium silicate with an SiO₂: Na₂O ratio of 2.0 is the most preferred silicate. Alkali metal persilicates are also suitable sources of silicate herein.

Preferred crystalline layered silicates for use herein have the general formula

NaMSiₓO₂ₓ₊₁.yH₂0

wherein M is sodium or hydrogen, x is a number from 1.9 to 4 and y is a number from 0 to 20. Crystalline layered sodium silicates of this type are disclosed in EP-A-0164514 and methods for their preparation are disclosed in DE-A-3417649 and DE-A-3742043. Herein, x in the general formula above preferably has a value of 2, 3 or 4 and is preferably 2. The most preferred material is δ-Na₂Si₂O₅, available from Hoechst AG as NaSKS-6.

The crystalline layered silicate material is preferably present in granular detergent compositions as a particulate in intimate admixture with a solid, water-soluble ionisable material. The solid, water-soluble ionisable material is selected from organic acids, organic and inorganic acid salts and mixtures thereof.

A preferred detergent composition herein comprises
(a) from 1% to 90% by weight of the composition of a surfactant system comprising an anionic surfactant and a cationic ester surfactant in accord with the present invention at a weight ratio of anionic to cationic ester surfactant of from 3:1 to 15:1; and
(b) from 1.5% to 95% by weight of the composition of an alkalinity system comprising alkaline salts selected from the group consisting of alkali metal or alkaline earth carbonate, bicarbonate, hydroxide or silicate, including crystalline layered silicate, salts and any mixtures thereof.

### Water-soluble builder compound

The detergent compositions herein preferably contain a water-soluble builder compound, typically present at a level of from 1 % to 80% by weight, preferably from 10% to 70% by weight, most preferably from 20% to 60% by weight of the composition.

Suitable water-soluble builder compounds include the water soluble monomeric polycarboxylates, or their acid forms, homo or copolymeric polycarboxylic acids or their salts in which the polycarboxylic acid comprises at least two carboxylic radicals separated from each other by not more that two carbon atoms, borates, phosphates, and mixtures of any of the foregoing.

The carboxylate or polycarboxylate builder can be momomeric or oligomeric in type although monomeric polycarboxylates are generally preferred for reasons of cost and performance.

Suitable carboxylates containing one carboxy group include the water soluble salts of lactic acid, glycolic acid and ether derivatives thereof. Polycarboxylates containing two carboxy groups include the water-soluble salts of succinic acid, malonic acid, (ethylenedioxy) diacetic acid, maleic acid, diglycolic acid, tartaric acid, tartronic acid and fumaric acid, as well as the ether carboxylates and the sulfinyl carboxylates. Polycarboxylates containing three carboxy groups include, in particular, water-soluble citrates, aconitrates and citraconates as well as succinate derivatives such as the carboxymethyloxysuccinates described in British Patent No. 1,379,241, lactoxysuccinates described in British Patent No. 1,389,732, and aminosuccinates described in Netherlands Application 7205873, and the oxypolycarboxylate materials such as 2-oxa-1,1,3-propane tricarboxylates described in British Patent No. 1,387,447.

Polycarboxylates containing four carboxy groups include oxydisuccinates disclosed in British Patent No. 1,261,829, 1,1,2,2-ethane tetracarboxylates, 1,1,3,3-propane tetracarboxylates and 1,1,2,3-propane tetracarboxylates. Polycarboxylates containing sulfo substituents include the sulfosuccinate derivatives disclosed in British Patent Nos. 1,398,421 and 1,398,422 and in U.S. Patent No. 3,936,448, and the sulfonated pyrolysed citrates described in British Patent No. 1,439,000. Preferred polycarboxylates are hydroxycarboxylates containing up to three carboxy groups per molecule, more particularly citrates.

The parent acids of the monomeric or oligomeric polycarboxylate chelating agents or mixtures thereof with their salts, e.g. citric acid or citrate/citric acid mixtures are also contemplated as useful builder components.

Borate builders, as well as builders containing borate-forming materials that can produce borate under detergent storage or wash conditions are useful water-soluble builders herein.

Suitable examples of water-soluble phosphate builders are the alkali metal tripolyphosphates, sodium, potassium and ammonium pyrophosphate, sodium and potassium and ammonium pyrophosphate, sodium and potassium orthophosphate, sodium polymeta/phosphate in which the degree of polymerization ranges from about 6 to 21, and salts of phytic acid.

### Partially soluble or insoluble builder compound

The detergent compositions herein may contain a partially soluble or insoluble builder compound, typically present at a level of from 1 % to 80% by weight, preferably from 10% to 70% by weight, most preferably from 20% to 60% weight of the composition.

Examples of largely water insoluble builders include the sodium aluminosilicates.

Suitable aluminosilicate zeolites have the unit cell formula Na_{z}[(AlO₂)_{z}(SiO₂)_{y}]. xH₂O wherein z and y are at least 6; the molar ratio of z to y is from 1.0 to 0.5 and x is at least 5, preferably from 7.5 to 276, more preferably from 10 to 264. The aluminosilicate material are in hydrated form and are preferably crystalline, containing from 10% to 28%, more preferably from 18 % to 22% water in bound form.

The aluminosilicate zeolites can be naturally occurring materials, but are preferably synthetically derived. Synthetic crystalline aluminosilicate ion exchange materials are available under the designations Zeolite A, Zeolite B, Zeolite P, Zeolite X, Zeolite HS and mixtures thereof. Zeolite A has the formula

Na ₁₂ [AlO₂) ₁₂ (SiO₂)₁₂]. xH₂O

wherein x is from 20 to 30, especially 27. Zeolite X has the formula Na₈₆ [(AlO₂)₈₆(SiO₂)₁₀₆]. 276 H₂O.

### Organic peroxyacid bleaching system

A preferred feature of detergent compositions herein is an organic peroxyacid bleaching system. In one preferred execution the bleaching system contains a hydrogen peroxide source and an organic peroxyacid bleach precursor compound. The production of the organic peroxyacid occurs by an in situ reaction of the precursor with a source of hydrogen peroxide. Preferred sources of hydrogen peroxide include inorganic perhydrate bleaches. In an alternative preferred execution a preformed organic peroxyacid is incorporated directly into the composition. Compositions containing mixtures of a hydrogen peroxide source and organic peroxyacid precursor in combination with a preformed organic peroxyacid are also envisaged.

### Inorganic perhydrate bleaches

Inorganic perhydrate salts are a preferred source of hydrogen peroxide. These salts are normally incorporated in the form of the alkali metal, preferably sodium salt at a level of from 1 % to 40% by weight, more preferably from 2% to 30% by weight and most preferably from 5% to 25 % by weight of the compositions.

Examples of inorganic perhydrate salts include perborate, percarbonate, perphosphate, persulfate and persilicate salts. The inorganic perhydrate salts are normally the alkali metal salts. The inorganic perhydrate salt may be included as the crystalline solid without additional protection. For certain perhydrate salts however, the preferred executions of such granular compositions utilize a coated form of the material which provides better storage stability for the perhydrate salt in the granular product. Suitable coatings comprise inorganic salts such as alkali metal silicate, carbonate or borate salts or mixtures thereof, or organic materials such as waxes, oils, or fatty soaps.

Sodium perborate is a preferred perhydrate salt and can be in the form of the monohydrate of nominal formula NaBO₂H₂O₂ or the tetrahydrate NaBO₂H₂O₂.3H₂O.

Alkali metal percarbonates, particularly sodium percarbonate are preferred perhydrates herein. Sodium percarbonate is an addition compound having a formula corresponding to 2Na₂CO₃.3H₂O₂, and is available commercially as a crystalline solid.

Potassium peroxymonopersulfate is another inorganic perhydrate salt of use in the detergent compositions herein.

### Peroxyacid bleach precursor

Peroxyacid bleach precursors are compounds which react with hydrogen peroxide in a perhydrolysis reaction to produce a peroxyacid. Generally peroxyacid bleach precursors may be represented as 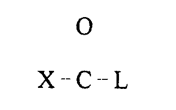 where L is a leaving group and X is essentially any functionality, such that on perhydroloysis the structure of the peroxyacid produced is

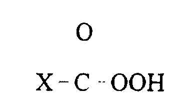

Peroxyacid bleach precursor compounds are preferably incorporated at a level of from 0.5% to 20% by weight, more preferably from 1% to 15% by weight, most preferably from 1.5% to 10% by weight of the detergent compositions.

Suitable peroxyacid bleach precursor compounds typically contain one or more N- or O-acyl groups, which precursors can be selected from a wide range of classes. Suitable classes include anhydrides, esters, imides, lactams and acylated derivatives of imidazoles and oximes. Examples of useful materials within these classes are disclosed in GB-A-1586789. Suitable esters are disclosed in GB-A-836988, 864798, 1147871, 2143231 and EP-A-0170386.

### Leaving groups

The leaving group, hereinafter L group, must be sufficiently reactive for the perhydrolysis reaction to occur within the optimum time frame (e.g., a wash cycle). However, if L is too reactive, this activator will be difficult to stabilize for use in a bleaching composition.

Preferred L groups are selected from the group consisting of: 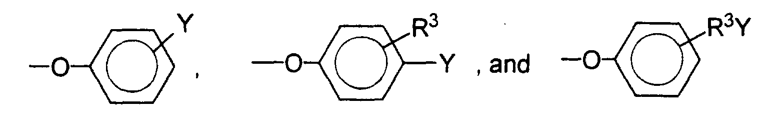 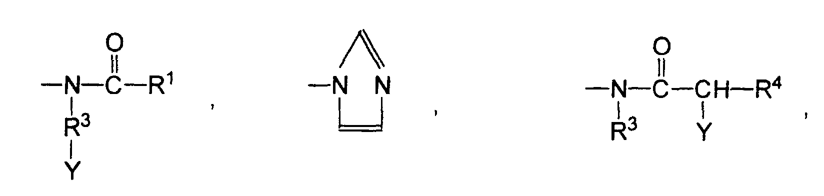 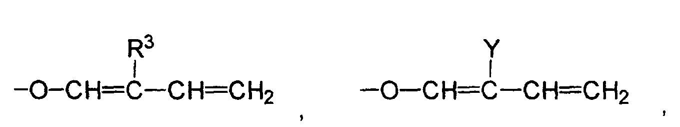 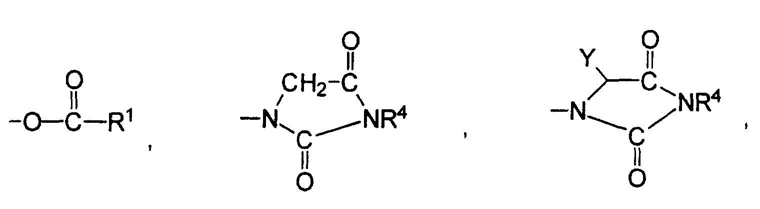 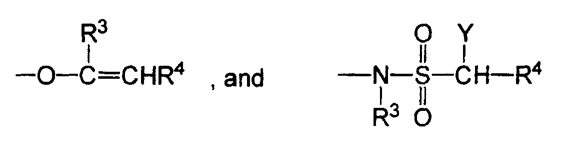 and mixtures thereof, wherein R¹ is an alkyl, aryl, or alkaryl group containing from 1 to 14 carbon atoms, R³ is an alkyl chain containing from 1 to 8 carbon atoms, R⁴ is H or R³, and Y is H or a solubilizing group. Any of R¹, R³ and R⁴ may be substituted by essentially any functional group including, for example alkyl, hydroxy, alkoxy, halogen, amine, nitrosyl, amide and ammonium or alkyl ammmonium groups

The preferred solubilizing groups are -SO₃⁻M⁺, -CO₂⁻M⁺, -SO₄⁻M⁺, -N⁺(R³)₄X⁻ and O<--N(R³)₃ and most preferably -SO₃⁻M⁺ and -CO₂⁻M⁺ wherein R is an alkyl chain containing from 1 to 4 carbon atoms, M is a cation which provides solubility to the bleach activator and X is an anion which provides solubility to the bleach activator. Preferably, M is an alkali metal, ammonium or substituted ammonium cation, with sodium and potassium being most preferred, and X is a halide, hydroxide, methylsulfate or acetate anion.

### Alkyl percarboxylic acid bleach precursors

Alkyl percarboxylic acid bleach precursors form percarboxylic acids on perhydrolysis. Preferred precursors of this type provide peracetic acid on perhydrolysis.

Preferred alkyl percarboxylic precursor compounds of the imide type include the N-,N,N¹N¹ tetra acetylated alkylene diamines wherein the alkylene group contains from 1 to 6 carbon atoms, particularly those compounds in which the alkylene group contains 1, 2 and 6 carbon atoms. Tetraacetyl ethylene diamine (TAED) is particularly preferred.

Other preferred alkyl percarboxylic acid precursors include sodium 3,5,5-tri-methyl hexanoyloxybenzene sulfonate (iso-NOBS), sodium nonanoyloxybenzene sulfonate (NOBS), sodium acetoxybenzene sulfonate (ABS) and pentaacetyl glucose.

### Amide substituted alkyl peroxyacid precursors

Amide substituted alkyl peroxyacid precursor compounds are suitable herein, including those of the following general formulae: 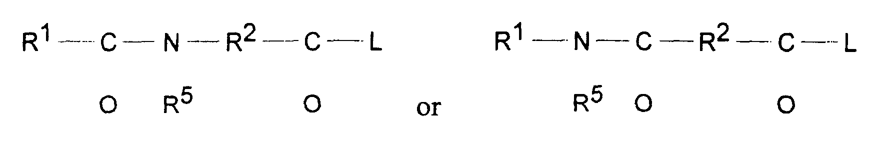 wherein R¹ is an alkyl group with from 1 to 14 carbon atoms, R² is an alkylene group containing from 1 to 14 carbon atoms, and R⁵ is H or an alkyl group containing 1 to 10 carbon atoms and L can be essentially any leaving group. Amide substituted bleach activator compounds of this type are described in EP-A-0170386.

### Perbenzoic acid precursor

Perbenzoic acid precursor compounds provide perbenzoic acid on perhydrolysis. Suitable O-acylated perbenzoic acid precursor compounds include the substituted and unsubstituted benzoyl oxybenzene sulfonates, and the benzoylation products of sorbitol, glucose, and all saccharides with benzoylating agents, and those of the imide type including N-benzoyl succinimide, tetrabenzoyl ethylene diamine and the N-benzoyl substituted ureas. Suitable imidazole type perbenzoic acid precursors include N-benzoyl imidazole and N-benzoyl benzimidazole. Other useful N-acyl group-containing perbenzoic acid precursors include N-benzoyl pyrrolidone, dibenzoyl taurine and benzoyl pyroglutamic acid.

### Cationic peroxyacid precursors

Cationic peroxyacid precursor compounds produce cationic peroxyacids on perhydrolysis.

Typically, cationic peroxyacid precursors are formed by substituting the peroxyacid part of a suitable peroxyacid precursor compound with a positively charged functional group, such as an ammonium or alkyl ammmonium group, preferably an ethyl or methyl ammonium group. Cationic peroxyacid precursors are typically present in the solid detergent compositions as a salt with a suitable anion, such as a halide ion.

The peroxyacid precursor compound to be so cationically substituted may be a perbenzoic acid, or substituted derivative thereof, precursor compound as described hereinbefore. Alternatively, the peroxyacid precursor compound may be an alkyl percarboxylic acid precursor compound or an amide substituted alkyl peroxyacid precursor as described hereinafter

Cationic peroxyacid precursors are described in U.S. Patents 4,904,406; 4,751,015; 4,988,451; 4,397,757; 5,269,962; 5,127,852; 5,093,022; 5,106,528; U.K. 1,382,594; EP 475,512, 458,396 and 284,292; and in JP 87-318,332.

Examples of preferred cationic peroxyacid precursors are described in UK Patent Application No. 9407944.9 and US Patent Application Nos. 08/298903, 08/298650, 08/298904 and 08/298906.

Suitable cationic peroxyacid precursors include any of the ammonium or alkyl ammonium substituted alkyl or benzoyl oxybenzene sulfonates, N-acylated caprolactams, and monobenzoyltetraacetyl glucose benzoyl peroxides. Preferred cationic peroxyacid precursors of the N-acylated caprolactam class include the trialkyl ammonium methylene benzoyl caprolactams and the trialkyl ammonium methylene alkyl caprolactams.

### Benzoxazin organic peroxyacid precursors

Also suitable are precursor compounds of the benzoxazin-type, as disclosed for example in EP-A-332,294 and EP-A-482,807, particularly those having the formula: 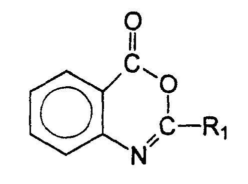 wherein R₁ is H, alkyl, alkaryl, aryl, or arylalkyl.

### Preformed organic peroxyacid

The organic peroxyacid bleaching system may contain, in addition to, or as an alternative to, an organic peroxyacid bleach precursor compound, a preformed organic peroxyacid , typically at a level of from 1 % to 15 % by weight, more preferably from 1 % to 10% by weight of the composition.

A preferred class of organic peroxyacid compounds are the amide substituted compounds of the following general formulae: 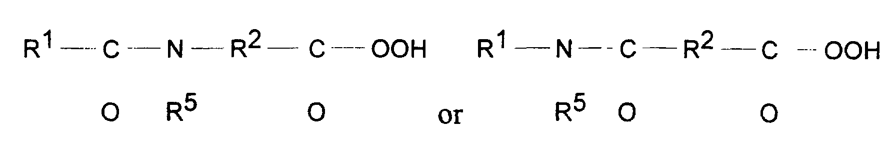 wherein R¹ is an alkyl, aryl or alkaryl group with from 1 to 14 carbon atoms, R² is an alkylene, arylene, and alkarylene group containing from 1 to 14 carbon atoms, and R⁵ is H or an alkyl, aryl, or alkaryl group containing 1 to 10 carbon atoms. Amide substituted organic peroxyacid compounds of this type are described in EP-A-0170386.

Other organic peroxyacids include diacyl and tetraacylperoxides, especially diperoxydodecanedioc acid, diperoxytetradecanedioc acid and diperoxyhexadecanedioc acid. Mono- and diperazelaic acid, mono- and diperbrassylic acid and N-phthaloylaminoperoxicaproic acid are also suitable herein.

### Bleach catalyst

The detergent compositions optionally contain a transition metal containing bleach catalyst. One suitable type of bleach catalyst is a catalyst system comprising a heavy metal cation of defined bleach catalytic activity, such as copper, iron or manganese cations, an auxiliary metal cation having little or no bleach catalytic activity, such as zinc or aluminum cations, and a sequestrant having defined stability constants for the catalytic and auxiliary metal cations, particularly ethylenediaminetetraacetic acid, ethylenediaminetetra(methylenephosphonic acid) and water-soluble salts thereof. Such catalysts are disclosed in U.S. Pat. 4,430,243.

Other types of bleach catalysts include the manganese-based complexes disclosed in U.S. Pat. 5,246,621 and U.S. Pat. 5,244,594. Preferred examples of these catalysts include Mn^{IV}₂(u-O)₃(1,4,7-trimethyl-1,4,7-triazacyclononane)₂-(PF₆)₂, Mn^{III}₂(u-O)₁(u-OAc)₂(1,4,7-trimethyl-1,4,7-triazacyclononane)₂-(ClO₄)₂, Mn^{IV}₄(u-O)₆(1,4,7-triazacyclononane)₄-(ClO₄)₂, Mn^{III}Mn^{IV}₄(u-O)₁(u-OAc)₂₋(1,4,7-trimethyl-1,4,7-triazacyclononane)₂-(ClO₄)₃, and mixtures thereof. Others are described in European patent application publication no. 549,272. Other ligands suitable for use herein include 1,5,9-trimethyl-1,5,9-triazacyclododecane, 2-methyl-1,4,7-triazacyclononane, 2-methyl-1,4,7-triazacyclononane, 1,2,4,7-tetramethyl-1,4,7-triazacyclononane, and mixtures thereof.

For examples of suitable bleach catalysts see U.S. Pat. 4,246,612 and U.S. Pat. 5,227,084. See also U.S. Pat. 5,194,416 which teaches mononuclear manganese (IV) complexes such as Mn(1,4,7-trimethyl-1,4,7-triazacyclononane)(OCH₃)₃-(PF₆). Still another type of bleach catalyst, as disclosed in U.S. Pat. 5,114,606, is a water-soluble complex of manganese (III), and/or (IV) with a ligand which is a non-carboxylate polyhydroxy compound having at least three consecutive C-OH groups. Other examples include binuclear Mn complexed with tetra-N-dentate and bi-N-dentate ligands, including N₄Mn^{III}(u-O)₂Mn^{IV}N₄)⁺and [Bipy₂Mn^{III}(u-O)₂Mn^{IV}bipy₂]-(ClO₄)₃.

Further suitable bleach catalysts are described, for example, in European patent application No. 408,131 (cobalt complex catalysts), European patent applications, publication nos. 384,503, and 306,089 (metalloporphyrin catalysts), U.S. 4,728,455 (manganese/multidentate ligand catalyst), U.S. 4,711,748 and European patent application, publication no. 224,952, (absorbed manganese on aluminosilicate catalyst), U.S. 4,601,845 (aluminosilicate support with manganese and zinc or magnesium salt), U.S. 4,626,373 (manganese/ligand catalyst), U.S. 4,119,557 (ferric complex catalyst), German Pat. specification 2,054,019 (cobalt chelant catalyst) Canadian 866,191 (transition metal-containing salts), U.S. 4,430,243 (chelants with manganese cations and non-catalytic metal cations), and U.S. 4,728,455 (manganese gluconate catalysts).

### Heavy metal ion sequestrant

The detergent compositions herein preferably contain as an optional component a heavy metal ion sequestrant. By heavy metal ion sequestrant it is meant herein components which act to sequester (chelate) heavy metal ions. These components may also have calcium and magnesium chelation capacity, but preferentially they show selectivity to binding heavy metal ions such as iron, manganese and copper.

Heavy metal ion sequestrants are generally present at a level of from 0.005% to 20%, preferably from 0.1 % to 10%, more preferably from 0.25% to 7.5% and most preferably from 0.5% to 5% by weight of the compositions.

Suitable heavy metal ion sequestrants for use herein include organic phosphonates, such as the amino alkylene poly (alkylene phosphonates), alkali metal ethane 1-hydroxy disphosphonates and nitrilo trimethylene phosphonates.

Preferred among the above species are diethylene triamine penta (methylene phosphonate), ethylene diamine tri (methylene phosphonate) hexamethylene diamine tetra (methylene phosphonate) and hydroxyethylene 1,1 diphosphonate.

Other suitable heavy metal ion sequestrant for use herein include nitrilotriacetic acid and polyaminocarboxylic acids such as ethylenediaminotetracetic acid, ethylenetriamine pentacetic acid, ethylenediamine disuccinic acid, ethylenediamine diglutaric acid, 2-hydroxypropylenediamine disuccinic acid or any salts thereof. Especially preferred is ethylenediamine-N,N'-disuccinic acid (EDDS) or the alkali metal, alkaline earth metal, ammonium, or substituted ammonium salts thereof, or mixtures thereof.

Other suitable heavy metal ion sequestrants for use herein are iminodiacetic acid derivatives such as 2-hydroxyethyl diacetic acid or glyceryl imino diacetic acid, described in EP-A-317,542 and EP-A-399,133. The iminodiacetic acid-N-2-hydroxypropyl sulfonic acid and aspartic acid N-carboxymethyl N-2-hydroxypropyl-3-sulfonic acid sequestrants described in EP-A-516,102 are also suitable herein. The β-alanine-N,N'-diacetic acid, aspartic acid-N,N'-diacetic acid, aspartic acid-N-monoacetic acid and iminodisuccinic acid sequestrants described in EP-A-509,382 are also suitable.

EP-A-476,257 describes suitable amino based sequestrants. EP-A-510,331 describes suitable sequestrants derived from collagen, keratin or casein. EP-A-528,859 describes a suitable alkyl iminodiacetic acid sequestrant. Dipicolinic acid and 2-phosphonobutane-1,2,4-tricarboxylic acid are alos suitable. Glycinamide-N,N'-disuccinic acid (GADS), ethylenediamine-N-N'-diglutaric acid (EDDG) and 2-hydroxypropylenediamine-N-N'-disuccinic acid (HPDDS) are also suitable.

### Enzyme

Another preferred ingredient useful in the detergent compositions is one or more additional enzymes.

Preferred additional enzymatic materials include the commercially available lipases, cutinases, amylases, neutral and alkaline proteases, esterases, cellulases, pectinases, lactases and peroxidases conventionally incorporated into detergent compositions. Suitable enzymes are discussed in US Patents 3,519,570 and 3,533,139.

Preferred commercially available protease enzymes include those sold under the tradenames Alcalase, Savinase, Primase, Durazym, and Esperase by Novo Industries A/S (Denmark), those sold under the tradename Maxatase, Maxacal and Maxapem by Gist-Brocades, those sold by Genencor International, and those sold under the tradename Opticlean and Optimase by Solvay Enzymes. Protease enzyme may be incorporated into the compositions in accordance with the invention at a level of from 0.0001 % to 4% active enzyme by weight of the composition.

Preferred amylases include, for example, α-amylases obtained from a special strain of B licheniformis, described in more detail in GB-1,269,839 (Novo). Preferred commercially available amylases include for example, those sold under the tradename Rapidase by Gist-Brocades, and those sold under the tradename Termamyl and BAN by Novo Industries A/S. Amylase enzyme may be incorporated into the composition in accordance with the invention at a level of from 0.0001% to 2% active enzyme by weight of the composition.

Lipolytic enzyme may be present at levels of active lipolytic enzyme of from 0.0001% to 2% by weight, preferably 0.001% to 1% by weight, most preferably from 0.001 % to 0.5% by weight of the compositions.

The lipase may be fungal or bacterial in origin being obtained, for example, from a lipase producing strain of Humicola sp., Thermomyces sp. or Pseudomonas sp. including Pseudomonas pseudoalcaligenes or Pseudomas fluorescens. Lipase from chemically or genetically modified mutants of these strains are also useful herein. A preferred lipase is derived from Pseudomonas pseudoalcaligenes, which is described in Granted European Patent, EP-B-0218272.

Another preferred lipase herein is obtained by cloning the gene from Humicola lanuginosa and expressing the gene in Aspergillus oryza, as host, as described in European Patent Application, EP-A-0258 068, which is commercially available from Novo Industri A/S, Bagsvaerd, Denmark, under the trade name Lipolase. This lipase is also described in U.S. Patent 4,810,414, Huge-Jensen et al, issued March 7, 1989.

### Organic polymeric compound

Organic polymeric compounds are preferred additional components of the detergent compositions herein, and are preferably present as components of any particulate components where they may act such as to bind the particulate component together. By organic polymeric compound it is meant herein essentially any polymeric organic compound commonly used as dispersants, and anti-redeposition and soil suspension agents in detergent compositions, including any of the high molecular weight organic polymeric compounds described as clay flocculating agents herein.

Organic polymeric compound is typically incorporated in the detergent compositions of the invention at a level of from 0.1 % to 30%, preferably from 0.5% to 15%, most preferably from 1% to 10% by weight of the compositions.

Examples of organic polymeric compounds include the water soluble organic homo- or co-polymeric polycarboxylic acids or their salts in which the polycarboxylic acid comprises at least two carboxyl radicals separated from each other by not more than two carbon atoms. Polymers of the latter type are disclosed in GB-A-1,596,756. Examples of such salts are polyacrylates of MWt 2000-5000 and their copolymers with maleic anhydride, such copolymers having a molecular weight of from 20,000 to 100,000, especially 40,000 to 80,000.

The polyamino compounds are useful herein including those derived from aspartic acid such as those disclosed in EP-A-305282, EP-A-305283 and EP-A-351629.

Terpolymers containing monomer units selected from maleic acid, acrylic acid, polyaspartic acid and vinyl alcohol, particularly those having an average molecular weight of from 5,000 to 10,000, are also suitable herein.

Other organic polymeric compounds suitable for incorporation in the detergent compositions herein include cellulose derivatives such as methylcellulose, carboxymethylcellulose, hydroxypropylmethylcellulose and hydroxyethylcellulose.

Further useful organic polymeric compounds are the polyethylene glycols, particularly those of molecular weight 1000-10000, more particularly 2000 to 8000 and most preferably about 4000.

### Suds suppressing system

The detergent compositions herein, when formulated for use in machine washing compositions, preferably comprise a suds suppressing system present at a level of from 0.01% to 15%, preferably from 0.05% to 10%, most preferably from 0.1 % to 5% by weight of the composition.

Suitable suds suppressing systems for use herein may comprise essentially any known antifoam compound, including, for example silicone antifoam compounds and 2-alkyl alcanol antifoam compounds.

By antifoam compound it is meant herein any compound or mixtures of compounds which act such as to depress the foaming or sudsing produced by a solution of a detergent composition, particularly in the presence of agitation of that solution.

Particularly preferred antifoam compounds for use herein are silicone antifoam compounds defined herein as any antifoam compound including a silicone component. Such silicone antifoam compounds also typically contain a silica component. The term "silicone" as used herein, and in general throughout the industry, encompasses a variety of relatively high molecular weight polymers containing siloxane units and hydrocarbyl group of various types. Preferred silicone antifoam compounds are the siloxanes, particularly the polydimethylsiloxanes having trimethylsilyl end blocking units.

Other suitable antifoam compounds include the monocarboxylic fatty acids and soluble salts thereof. These materials are described in US Patent 2,954,347, issued September 27, 1960 to Wayne St. John. The monocarboxylic fatty acids, and salts thereof, for use as suds suppressor typically have hydrocarbyl chains of 10 to 24 carbon atoms, preferably 12 to 18 carbon atoms. Suitable salts include the alkali metal salts such as sodium, potassium, and lithium salts, and ammonium and alkanolammonium salts.

Other suitable antifoam compounds include, for example, high molecular weight fatty esters (e.g. fatty acid triglycerides), fatty acid esters of monovalent alcohols, aliphatic C₁₈-C₄₀ ketones (e.g. stearone) N-alkylated amino triazines such as tri- to hexa-alkylmelamines or di- to tetra alkyldiamine chlortriazines formed as products of cyanuric chloride with two or three moles of a primary or secondary amine containing 1 to 24 carbon atoms, propylene oxide, bis stearic acid amide and monostearyl di-alkali metal (e.g. sodium, potassium, lithium) phosphates and phosphate esters.

A preferred suds suppressing system comprises
(a) antifoam compound, preferably silicone antifoam compound, most preferably a silicone antifoam compound comprising in combination
   (i) polydimethyl siloxane, at a level of from 50% to 99%, preferably 75 % to 95 % by weight of the silicone antifoam compound; and
   (ii) silica, at a level of from 1 % to 50%, preferably 5% to 25% by weight of the silicone/silica antifoam compound;
   wherein said silica/silicone antifoam compound is incorporated at a level of from 5% to 50%, preferably 10% to 40% by weight;
(b) a dispersant compound, most preferably comprising a silicone glycol rake copolymer with a polyoxyalkylene content of 72-78 % and an ethylene oxide to propylene oxide ratio of from 1:0.9 to 1:1.1, at a level of from 0.5% to 10%, preferably 1% to 10% by weight; a particularly preferred silicone glycol rake copolymer of this type is DCO544, commercially available from DOW Corning under the tradename DCO544;
(c) an inert carrier fluid compound, most preferably comprising a C₁₆-C₁₈ ethoxylated alcohol with a degree of ethoxylation of from 5 to 50, preferably 8 to 15, at a level of from 5 % to 80%, preferably 10% to 70%, by weight;

A highly preferred particulate suds suppressing system is described in EP-A-0210731 and comprises a silicone antifoam compound and an organic carrier material having a melting point in the range 50°C to 85°C, wherein the organic carrier material comprises a monoester of glycerol and a fatty acid having a carbon chain containing from 12 to 20 carbon atoms. EP-A-0210721 discloses other preferred particulate suds suppressing systems wherein the organic carrier material is a fatty acid or alcohol having a carbon chain containing from 12 to 20 carbon atoms, or a mixture thereof, with a melting point of from 45°C to 80°C.

### Clay softening system

The detergent compositions may contain a clay softening system comprising a clay mineral compound and optionally a clay flocculating agent.

The clay mineral compound is preferably a smectite clay compound. Smectite clays are disclosed in the US Patents No.s 3,862,058, 3,948,790, 3,954,632 and 4,062,647. European Patents No.s EP-A-299,575 and EP-A-313,146 in the name of the Procter and Gamble Company describe suitable organic polymeric clay flocculating agents.

### Polymeric dye transfer inhibiting agents

The detergent compositions herein may also comprise from 0.01 % to 10 %, preferably from 0.05% to 0.5% by weight of polymeric dye transfer inhibiting agents.

The polymeric dye transfer inhibiting agents are preferably selected from polyamine N-oxide polymers, copolymers of N-vinylpyrrolidone and N-vinylimidazole, polyvinylpyrrolidonepolymers or combinations thereof.

### a) Polyamine N-oxide polymers

Polyamine N-oxide polymers suitable for use herein contain units having the following structure formula : 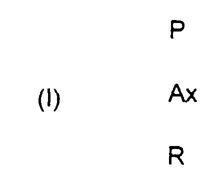 wherein P is a polymerisable unit, and 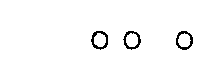 A is NC, CO, C, -O-, -S-, -N-; x is O or 1;

R are aliphatic, ethoxylated aliphatics, aromatic, heterocyclic or alicyclic groups or any combination thereof whereto the nitrogen of the N-O group can be attached or wherein the nitrogen of the N-O group is part of these groups.

The N-O group can be represented by the following general structures : 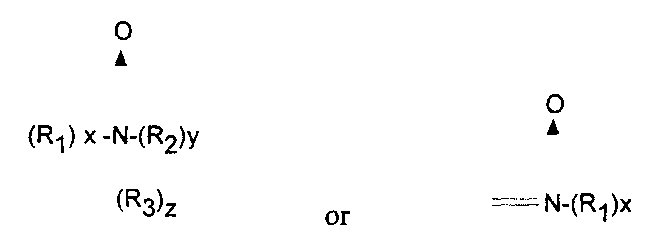 wherein R1, R2, and R3 are aliphatic groups, aromatic, heterocyclic or alicyclic groups or combinations thereof, x or/and y or/and z is 0 or 1 and wherein the nitrogen of the N-O group can be attached or wherein the nitrogen of the N-O group forms part of these groups. The N-O group can be part of the polymerisable unit (P) or can be attached to the polymeric backbone or a combination of both.

Suitable polyamine N-oxides wherein the N-O group forms part of the polymerisable unit comprise polyamine N-oxides wherein R is selected from aliphatic, aromatic, alicyclic or heterocyclic groups. One class of said polyamine N-oxides comprises the group of polyamine N-oxides wherein the nitrogen of the N-O group forms part of the R-group. Preferred polyamine N-oxides are those wherein R is a heterocyclic group such as pyrridine, pyrrole, imidazole, pyrrolidine, piperidine, quinoline, acridine and derivatives thereof.

Other suitable polyamine N-oxides are the polyamine oxides whereto the N-O group is attached to the polymerisable unit. A preferred class of these polyamine N-oxides comprises the polyamine N-oxides having the general formula (I) wherein R is an aromatic,heterocyclic or alicyclic groups wherein the nitrogen of the N-O functional group is part of said R group. Examples of these classes are polyamine oxides wherein R is a heterocyclic compound such as pyrridine, pyrrole, imidazole and derivatives thereof.

The polyamine N-oxides can be obtained in almost any degree of polymerisation. The degree of polymerisation is not critical provided the material has the desired water-solubility and dye-suspending power. Typically, the average molecular weight is within the range of 500 to 1000,000.

### b) Copolymers of N-vinylpyrrolidone and N-vinylimidazole

Suitable herein are coploymers of N-vinylimidazole and N-vinylpyrrolidone having an average molecular weight range of from 5,000 to 50,000. The preferred copolymers have a molar ratio of N-vinylimidazole to N-vinylpyrrolidone from 1 to 0.2.

### c) Polyvinylpyrrolidone

The detergent compositions herein may also utilize polyvinylpyrrolidone ("PVP") having an average molecular weight of from 2,500 to 400,000. Suitable polyvinylpyrrolidones are commercially vailable from ISP Corporation, New York, NY and Montreal, Canada under the product names PVP K-15 (viscosity molecular weight of 10,000), PVP K-30 (average molecular weight of 40,000), PVP K-60 (average molecular weight of 160,000), and PVP K-90 (average molecular weight of 360,000). PVP K-15 is also available from ISP Corporation. Other suitable polyvinylpyrrolidones which are commercially available from BASF Cooperation include Sokalan HP 165 and Sokalan HP 12.

### d) Polyvinyloxazolidone

The detergent compositions herein may also utilize polyvinyloxazolidones as polymeric dye transfer inhibiting agents. Said polyvinyloxazolidones have an average molecular weight of from 2,500 to 400,000.

### e) Polyvinylimidazole

The detergent compositions herein may also utilize polyvinylimidazole as polymeric dye transfer inhibiting agent. Said polyvinylimidazoles preferably have an average molecular weight of from 2,500 to 400,000.

### Optical brightener

The detergent compositions herein also optionally contain from about 0.005 % to 5 % by weight of certain types of hydrophilic optical brighteners.

Hydrophilic optical brighteners useful herein include those having the structural formula: 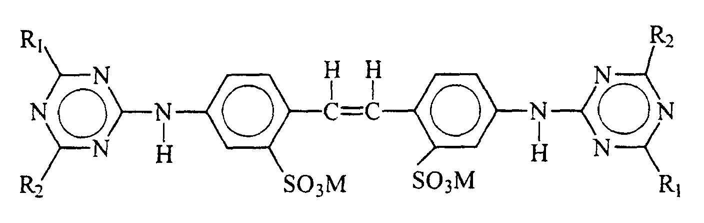 wherein R₁ is selected from anilino, N-2-bis-hydroxyethyl and NH-2-hydroxyethyl; R₂ is selected from N-2-bis-hydroxyethyl, N-2-hydroxyethyl-N-methylamino, morphilino, chloro and amino; and M is a salt-forming cation such as sodium or potassium.

When in the above formula, R₁ is anilino, R₂ is N-2-bis-hydroxyethyl and M is a cation such as sodium, the brightener is 4,4',-bis[(4-anilino-6-(N-2-bis-hydroxyethyl)-s-triazine-2-yl)amino]-2,2'-stilbenedisulfonic acid and disodium salt. This particular brightener species is commercially marketed under the tradename Tinopal-UNPA-GX by Ciba-Geigy Corporation. Tinopal-UNPA-GX is the preferred hydrophilic optical brightener useful in the detergent compositions herein.

When in the above formula, R₁ is anilino, R₂ is N-2-hydroxyethyl-N-2-methylamino and M is a cation such as sodium, the brightener is 4,4'-bis[(4-anilino-6-(N-2-hydroxyethyl-N-methylamino)-s-triazine-2-yl)amino]2,2'-stilbenedisulfonic acid disodium salt. This particular brightener species is commercially marketed under the tradename Tinopal 5BM-GX by Ciba-Geigy Corporation.

When in the above formula, R₁ is anilino, R₂ is morphilino and M is a cation such as sodium, the brightener is 4,4'-bis[(4-anilino-6-morphilino-s-triazine-2-yl)amino]2,2'-stilbenedisulfonic acid, sodium salt. This particular brightener species is commercially marketed under the tradename Tinopal AMS-GX by Ciba Geigy Corporation.

### Cationic fabric softening agents

Cationic fabric softening agents can also be incorporated into the detergent compositions herein. Suitable cationic fabric softening agents include the water insoluble tertiary amines or dilong chain amide materials as disclosed in GB-A-1 514 276 and EP-B-0 011 340.

Cationic fabric softening agents are typically incorporated at total levels of from 0.5% to 15% by weight, normally from 1 % to 5% by weight. Other optional ingredients

Other optional ingredients suitable for inclusion in the detergent compositions herein include perfumes, colours and filler salts, with sodium sulfate being a preferred filler salt.

### pH of the compositions

The present detergent compositions preferably have a pH measured as a 1 % solution in distilled water of at least 10.0, preferably from 10.0 to 12.5, most preferably from 10.5 to 12.0.

### Form of the compositions

The detergent compositions herein can take a variety of physical forms including granular, tablet, bar and liquid forms. The compositions are particularly the so-called concentrated granular detergent compositions adapted to be added to a washing machine by means of a dispensing device placed in the machine drum with the soiled fabric load.

The mean particle size of the components of granular compositions in accordance with the invention should preferably be such that no more that 5 % of particles are greater than 1.7mm in diameter and not more than 5% of particles are less than 0.15mm in diameter.

The term mean particle size as defined herein is calculated by sieving a sample of the composition into a number of fractions (typically 5 fractions) on a series of Tyler sieves. The weight fractions thereby obtained are plotted against the aperture size of the sieves. The mean particle size is taken to be the aperture size through which 50% by weight of the sample would pass.

The bulk density of granular detergent compositions herein typically have a bulk density of at least 600 g/litre, more preferably from 650 g/litre to 1200 g/litre.Bulk density is measured by means of a simple funnel and cup device consisting of a conical funnel moulded rigidly on a base and provided with a flap valve at its lower extremity to allow the contents of the funnel to be emptied into an axially aligned cylindrical cup disposed below the funnel. The funnel is 130 mm high and has internal diameters of 130 mm and 40 mm at its respective upper and lower extremities. It is mounted so that the lower extremity is 140 mm above the upper surface of the base. The cup has an overall height of 90 mm, an internal height of 87 mm and an internal diameter of 84 mm. Its nominal volume is 500 ml.

To carry out a measurement, the funnel is filled with powder by hand pouring, the flap valve is opened and powder allowed to overfill the cup. The filled cup is removed from the frame and excess powder removed from the cup by passing a straight edged implement eg; a knife, across its upper edge. The filled cup is then weighed and the value obtained for the weight of powder doubled to provide a bulk density in g/litre. Replicate measurements are made as required.

### Surfactant agglomerate particles

The surfactant system herein is preferably present in granular compositions in the form of surfactant agglomerate particles, which may take the form of flakes, prills, marumes, noodles, ribbons, but preferably take the form of granules. The most preferred way to process the particles is by agglomerating powders (e.g. aluminosilicate, carbonate) with high active surfactant pastes and to control the particle size of the resultant agglomerates within specified limits. Such a process involves mixing an effective amount of powder with a high active surfactant paste in one or more agglomerators such as a pan agglomerator, a Z-blade mixer or more preferably an in-line mixer such as those manufactured by Schugi (Holland) BV, 29 Chroomstraat 8211 AS, Lelystad, Netherlands, and Gebruder Lodige Maschinenbau GmbH, D-4790 Paderborn 1, Elsenerstrasse 7-9, Postfach 2050, Germany. Most preferably a high shear mixer is used, such as a Lodige CB (Trade Name).

A high active surfactant paste comprising from 50% by weight to 95 % by weight, preferably 70% by weight to 85% by weight of surfactant is typically used. The paste may be pumped into the agglomerator at a temperature high enough to maintain a pumpable viscosity, but low enough to avoid degradation of the anionic surfactants used. An operating temperature of the paste of 50°C to 80°C is typical.

### Laundry washing method

Machine laundry methods herein typically comprise treating soiled laundry with an aqueous wash solution in a washing machine having dissolved or dispensed therein an effective amount of a machine laundry detergent composition in accord with the invention. By an effective amount of the detergent composition it is meant from 40g to 300g of product dissolved or dispersed in a wash solution of volume from 5 to 65 litres, as are typical product dosages and wash solution volumes commonly employed in conventional machine laundry methods.

In a preferred use aspect a dispensing device is employed in the washing method. The dispensing device is charged with the detergent product, and is used to introduce the product directly into the drum of the washing machine before the commencement of the wash cycle. Its volume capacity should be such as to be able to contain sufficient detergent product as would normally be used in the washing method.

Once the washing machine has been loaded with laundry the dispensing device containing the detergent product is placed inside the drum. At the commencement of the wash cycle of the washing machine water is introduced into the drum and the drum periodically rotates. The design of the dispensing device should be such that it permits containment of the dry detergent product but then allows release of this product during the wash cycle in response to its agitation as the drum rotates and also as a result of its contact with the wash water.

To allow for release of the detergent product during the wash the device may possess a number of openings through which the product may pass. Alternatively, the device may be made of a material which is permeable to liquid but impermeable to the solid product, which will allow release of dissolved product. Preferably, the detergent product will be rapidly released at the start of the wash cycle thereby providing transient localised high concentrations of product in the drum of the washing machine at this stage of the wash cycle.

Preferred dispensing devices are reusable and are designed in such a way that container integrity is maintained in both the dry state and during the wash cycle. Especially preferred dispensing devices for use with the composition of the invention have been described in the following patents; GB-B-2, 157, 717, GB-B-2, 157, 718, EP-A-0201376, EP-A-0288345 and EP-A-0288346. An article by J.Bland published in Manufacturing Chemist, November 1989, pages 41-46 also describes especially preferred dispensing devices for use with granular laundry products which are of a type commonly know as the "granulette". Another preferred dispensing device for use with the detergent compositions herein is disclosed in PCT Patent Application No. WO94/11562.

Especially preferred dispensing devices are disclosed in European Patent Application Publication Nos. 0343069 & 0343070. The latter Application discloses a device comprising a flexible sheath in the form of a bag extending from a support ring defining an orifice, the orifice being adapted to admit to the bag sufficient product for one washing cycle in a washing process. A portion of the washing medium flows through the orifice into the bag, dissolves the product, and the solution then passes outwardly through the orifice into the washing medium. The support ring is provided with a masking arrangemnt to prevent egress of wetted, undissolved, product, this arrangement typically comprising radially extending walls extending from a central boss in a spoked wheel configuration, or a similar structure in which the walls have a helical form.

Alternatively, the dispensing device may be a flexible container, such as a bag or pouch. The bag may be of fibrous construction coated with a water impermeable protective material so as to retain the contents, such as is disclosed in European published Patent Application No. 0018678. Alternatively it may be formed of a water-insoluble synthetic polymeric material provided with an edge seal or closure designed to rupture in aqueous media as disclosed in European published Patent Application Nos. 0011500, 0011501, 0011502, and 0011968. A convenient form of water frangible closure comprises a water soluble adhesive disposed along and sealing one edge of a pouch formed of a water impermeable polymeric film such as polyethylene or polypropylene.

### Packaging for the compositions

Commercially marketed executions of the bleaching compositions can be packaged in any suitable container including those constructed from paper, cardboard, plastic materials and any suitable laminates. A preferred packaging execution is described in European Application No. 94921505.7.

### Abbreviations used in Examples

In the detergent compositions, the abbreviated component identifications have the following meanings:
- LAS :: Sodium linear C₁₂ alkyl benzene sulfonate
- TAS :: Sodium tallow alkyl sulfate
- C45AS :: Sodium C₁₄-C₁₅ linear alkyl sulfate
- CxyEzS :: Sodium C₁ₓ-C_{1y} branched alkyl sulfate condensed with z moles of ethylene oxide
- C45E7 :: A C₁₄₋₁₅ predominantly linear primary alcohol condensed with an average of 7 moles of ethylene oxide
- C25E3 :: A C₁₂₋₁₅ branched primary alcohol condensed with an average of 3 moles of ethylene oxide
- C25E5 :: A C₁₂₋₁₅ branched primary alcohol condensed with an average of 5 moles of ethylene oxide
- CEQ :: R₁COOCH₂CH₂CH₂.N⁺(CH₃)₃ with R₁ = C₁₁-C₁₃
- QAS :: R₂.N⁺(CH₃)₂(C₂H₄OH) with R₂ = C₁₂ - C₁₄
- Soap :: Sodium linear alkyl carboxylate derived from an 80/20 mixture of tallow and coconut oils.
- TFAA :: C₁₆-C₁₈ alkyl N-methyl glucamide
- TPKFA :: C12-C14 topped whole cut fatty acids
- STPP :: Anhydrous sodium tripolyphosphate
- Zeolite A :: Hydrated Sodium Aluminosilicate of formula Na₁₂(A10₂SiO₂)₁₂. 27H₂O having a primary particle size in the range from 0.1 to 10 micrometers
- NaSKS-6 :: Crystalline layered silicate of formula δ -Na₂Si₂O₅
- Citric acid :: Anhydrous citric acid
- Carbonate :: Anhydrous sodium carbonate with a particle size between 200µm and 900µm
- Bicarbonate :: Anhydrous sodium bicarbonate with a particle size distribution between 400µm and 1200µm
- Silicate :: Amorphous Sodium Silicate (SiO₂:Na₂O; 2.0 ratio)
- Sodium sulfate :: Anhydrous sodium sulfate
- Citrate :: Tri-sodium citrate dihydrate of activity 86.4% with a particle size distribution between 425µm and 850 µm
- MA/AA :: Copolymer of 1:4 maleic/acrylic acid, average molecular weight about 70,000.
- CMC :: Sodium carboxymethyl cellulose
- Protease :: Proteolytic enzyme of activity 4KNPU/g sold by NOVO Industries A/S under the tradename Savinase
- Alcalase :: Proteolytic enzyme of activity 3AU/g sold by NOVO Industries A/S
- Cellulase :: Cellulytic enzyme of activity 1000 CEVU/g sold by NOVO Industries A/S under the tradename Carezyme
- Amylase :: Amylolytic enzyme of activity 60KNU/g sold by NOVO Industries A/S under the tradename Termamyl 60T
- Lipase :: Lipolytic enzyme of activity 100kLU/g sold by NOVO Industries A/S under the tradename Lipolase
- Endolase :: Endoglunase enzyme of activity 3000 CEVU/g sold by NOVO Industries A/S
- PB4 :: Sodium perborate tetrahydrate of nominal formula NaBO₂.3H₂O.H₂O₂
- PB1 :: Anhydrous sodium perborate monohydrate bleach of nominal formula NaBO₂.H₂O₂
- Percarbonate :: Sodium Percarbonate of nominal formula 2Na₂CO₃.3H₂O₂
- NOBS :: Nonanoyloxybenzene sulfonate in the form of the sodium salt.
- TAED :: Tetraacetylethylenediamine
- DTPMP :: Diethylene triamine penta (methylene phosphonate), marketed by Monsanto under the Trade name Dequest 2060
- Photoactivated :: Sulfonated Zinc Phthlocyanine encapsulated in bleach dextrin soluble polymer
- Brightener 1 :: Disodium 4,4'-bis(2-sulphostyryl)biphenyl
- Brightener 2 :: Disodium 4,4'-bis(4-anilino-6-morpholino-1.3.5-triazin-2-yl)amino) stilbene-2:2'-disulfonate.
- HEDP :: 1,1-hydroxyethane diphosphonic acid
- PVNO :: Polyvinylpyridine N-oxide
- PVPVI :: Copolymer of polyvinylpyrolidone and vinylimidazole
- SRP 1 :: Sulfobenzoyl end capped esters with oxyethylene oxy and terephtaloyl backbone
- SRP 2 :: Diethoxylated poly (1, 2 propylene terephtalate) short block polymer
- Silicone antifoam :: Polydimethylsiloxane foam controller with siloxane-oxyalkylene copolymer as dispersing agent with a ratio of said foam controller to said dispersing agent of 10:1 to 100:1.
- Alkalinity :: % weight equivalent of NaOH, as obtained using the alkalinity release test method described herein.

In the following Examples all levels are quoted as % by weight of the composition:

### Example 1

Into a 500 ml conical flask fitted with a pressure-equalizing funnel, a magnetic stirrer and a drying tube was placed 3-dimethylamino-1-propanol (20.16 g, 0.195 mol) and triethylamine (21.75g, 0.215 mol) in 350 ml ether solvent. Dodecanoyl chloride (42.82g, 0.196 mol) was added dropwise via the pressure-equalizing funnel over a 1 hour period whilst keeping the reaction temperature below 30 °C with an ice bath. After addition of dodecanoyl chloride, the reaction was allowed to stir overnight at room temperature.

The reaction mixture was then purified by washing with saturated sodium bicarbonate (2x50ml) and drying the ether layer over anhydrous magnesium sulfate. After filtering to get rid of magnesium sulfate, the solvent was removed by rotary evaporation to give a light yellow ester amine product (23.50g, 42.1% yield).

Into a 250 ml round-bottomed flask fitted with dry-ice condenser / drying tube and magnetic stirrer and cooled with a dry-ice acetone bath was added the above ester amine product (23.00g ,0.081 mol) in 100 ml ether solvent. The reaction mixture was cooled to -10 °C and methylbromide (38.33g, 0.40 mol) was added via a graduated cylinder to the reaction. The reaction was kept between -10 °C and 0 °C for 1.5 hours and then allowed to cool to room temperature. After standing overnight, the solid product was transferred from the reaction flask with 500 ml ether and filtered. The white solid was placed in an evaporating dish and dried overnight in a vacuum dessicator over phosphorus pentoxide to give the desired cationic ester product (26.18g, 85.4% yield).

### Example 2

The following laundry detergent compositions A to F were prepared in accord with the invention:

| | A | B | C | D | E | F |
|---|---|---|---|---|---|---|
| LAS | 8.0 | 8.0 | 8.0 | 8.0 | 8.0 | 8.0 |
| C25E3 | 3.4 | 3.4 | 5.4 | 3.4 | 2.4 | 3.4 |
| CEQ | 2.0 | 0.8 | 1.0 | 1.5 | 0.8 | 0.8 |
| QAS | - | - | 0.8 | - | - | 0.4 |
| Zeolite A | 18.1 | 18.1 | 18.1 | 18.1 | 18.1 | 18.1 |
| Carbonate | 13.0 | 13.0 | 13.0 | 27.0 | 27.0 | 27.0 |
| Silicate | 1.4 | 1.4 | 1.4 | 3.0 | 3.0 | 3.0 |
| Sodium sulfate | 26.1 | 26.1 | 26.1 | 26.1 | 26.1 | 26.1 |
| PB4 | 9.0 | 9.0 | 9.0 | 9.0 | 9.0 | 9.0 |
| TAED | 1.5 | 1.5. | 1.5 | 1.5 | 1.5 | 1.5 |
| DETPMP | 0.25 | 0.25 | 0.25 | 0.25 | 0.25 | 0.25 |
| HEDP | 0.3 | 0.3 | 0.3 | 0.3 | 0.3 | 0.3 |
| Protease | 0.26 | 0.26 | 0.26 | 0.26 | 0.26 | 0.26 |
| Amylase | 0.1 | 0.1 | 0.1 | 0.1 | 0.1 | 0.1 |
| MA/AA | 0.3 | 0.3 | 0.3 | 0.3 | 0.3 | 0.3 |
| CMC | 0.2 | 0.2 | 0.2 | 0.2 | 0.2 | 0.2 |
| Photoactivated bleach (ppm) | 15 ppm | 15 ppm | 15 ppm | 15 ppm | 15 ppm | 15 ppm |
| Brightener 1 | 0.09 | 0.09 | 0.09 | 0.09 | 0.09 | 0.09 |
| Perfume | 0.3 | 0.3 | 0.3 | 0.3 | 0.3 | 0.3 |
| Silicone antifoam | 0.5 | 0.5 | 0.5 | 0.5 | 0.5 | 0.5 |
| Misc/minors to 100% | | | | | | |
| | | | | | | |
| Density in g/litre | 630 | 670 | 670 | 500 | 670 | 670 |
| Alkalinity | 6.8 | 6.8 | 6.8 | 18.5 | 18.5 | 18.5 |

### Example 3

The following granular laundry detergent compositions G to I of bulk density 750 g/litre were prepared in accord with the invention:

| | G | H | I |
|---|---|---|---|
| LAS | 5.25 | 5.61 | 4.76 |
| TAS | 1.25 | 1.86 | 1.57 |
| C45AS | - | 2.24 | 3.89 |
| C25AE3S | - | 0.76 | 1.18 |
| C45E7 | 3.25 | - | 5.0 |
| C25E3 | - | 5.5 | - |
| CEQ | 0.8 | 2.0 | 2.0 |
| STPP | 10.7 | - | - |
| Zeolite A | - | 19.5 | 19.5 |
| NaSKS-6/citric acid (79:21) | - | 10.6 | 10.6 |
| Carbonate | 16.1 | 21.4 | 21.4 |
| Bicarbonate | - | 2.0 | 2.0 |
| Silicate | 6.8 | - | - |
| Sodium sulfate | 39.8 | - | 14.3 |
| PB4 | 5.0 | 12.7 | - |
| TAED | 0.5 | 3.1 | - |
| DETPMP | 0.25 | 0.2 | 0.2 |
| HEDP | - | 0.3 | 0.3 |
| Protease | 0.26 | 0.85 | 0.85 |
| Lipase | 0.15 | 0.15 | 0.15 |
| Cellulase | 0.28 | 0.28 | 0.28 |
| Amylase | 0.1 | 0.1 | 0.1 |
| MA/AA | 0.8 | 1.6 | 1.6 |
| CMC | 0.2 | 0.4 | 0.4 |
| Photoactivated bleach (ppm) | 15 ppm | 27 ppm | 27 ppm |
| Brightener 1 | 0.08 | 0.19 | 0.19 |
| Brightener 2 | - | 0.04 | 0.04 |
| Perfume | 0.3 | 0.3 | 0.3 |
| Silicone antifoam | 0.5 | 2.4 | 2.4 |
| Minors/misc to 100% | | | |

### Example 4

The following detergent formulations, in accord with the present invention were prepared, where J is a phosphorus-containing detergent composition, K is a zeolite-containing detergent composition and L is a compact detergent composition:

| | | J | K | L |
|---|---|---|---|---|
| Blown Powder | | | | |
| | STPP | 14.0 | - | 14.0 |
| | Zeolite A | - | 20.0 | - |
| | C45AS | 9.0 | 6.0 | 8.0 |
| | MA/AA | 2.0 | 4.0 | 2.0 |
| | LAS | 6.0 | 8.0 | 9.0 |
| | TAS | 2.0 | - | - |
| | CEQ | 1.5 | 3.0 | 3.5 |
| | Silicate | 7.0 | 8.0 | 8.0 |
| | CMC | 1.0 | 1.0 | 0.5 |
| | Brightener 2 | 0.2 | 0.2 | 0.2 |
| | Soap | 1.0 | 1.0 | 1.0 |
| | DTPMP | 0.4 | 0.4 | 0.2 |
| Spray On | | | | |
| | C45E7 | 2.5 | 2.5 | 2.0 |
| | C25E3 | 2.5 | 2.5 | 2.0 |
| | Silicone antifoam | 0.3 | 0.3 | 0.3 |
| | Perfume | 0.3 | 0.3 | 0.3 |
| Dry additives | | | | |
| | Carbonate | 26.0 | 23.0 | 25.0 |
| | PB4 | 18.0 | 18.0 | 10 |
| | PB1 | 4.0 | 4.0 | 0 |
| | TAED | 3.0 | 3.0 | 1.0 |
| | Photoactivated bleach | 0.02 | 0.02 | 0.02 |
| | Protease | 1.0 | 1.0 | 1.0 |
| | Lipase | 0.4 | 0.4 | 0.4 |
| | Amylase | 0.25 | 0.30 | 0.15 |
| | Dry mixed sodium sulfate | 3.0 | 3.0 | 5.0 |
| | Balance (Moisture & Miscellaneous) | 100.0 | 100.0 | 100.0 |
| Density (g/litre) | | 630 | 670 | 670 |

### Example 5

The following nil bleach-containing detergent formulations of particular use in the washing of colored clothing, in accord with the present invention were prepared:

| | | M | N | O |
|---|---|---|---|---|
| Blown Powder | | | | |
| | Zeolite A | 15.0 | 15.0 | - |
| | Sodium sulfate | 0.0 | 5.0 | - |
| | LAS | 3.0 | 3.0 | - |
| | CEQ | 2.0 | 1.5 | 1.3 |
| | DTPMP | 0.4 | 0.5 | - |
| | CMC | 0.4 | 0.4 | - |
| | MA/AA | 4.0 | 4.0 | - |
| Agglomerates | | | | |
| | C45AS | - | - | 11.0 |
| | LAS | 6.0 | 5.0 | - |
| | TAS | 3.0 | 2.0 | - |
| | Silicate | 4.0 | 4.0 | - |
| | Zeolite A | 10.0 | 15.0 | 13.0 |
| | CMC | - | - | 0.5 |
| | MA/AA | - | - | 2.0 |
| | Carbonate | 9.0 | 7.0 | 7.0 |
| Spray On | | | | |
| | Perfume | 0.3 | 0.3 | 0.5 |
| | C45E7 | 4.0 | 4.0 | 4.0 |
| | C25E3 | 2.0 | 2.0 | 2.0 |
| Dry additives | | | | |
| | MA/AA | - | - | 3.0 |
| | NaSKS-6 | - | - | 12.0 |
| | Citrate | 10.0 | - | 8.0 |
| | Bicarbonate | 7.0 | 3.0 | 5.0 |
| | Carbonate | 8.0 | 5.0 | 7.0 |
| | PVPVI/PVNO | 0.5 | 0.5 | 0.5 |
| | Alcalase | 0.5 | 0.3 | 0.9 |
| | Lipase | 0.4 | 0.4 | 0.4 |
| | Amylase | 0.6 | 0.6 | 0.6 |
| | Cellulase | 0.6 | 0.6 | 0.6 |
| | Silicone antifoam | 5.0 | 5.0 | 5.0 |
| Dry additives | | | | |
| | Sodium sulfate | 0.0 | 9.0 | 0.0 |
| | Balance (Moisture and Miscellaneous) | 100.0 | 100.0 | 100.0 |
| Density (g/litre) | | 700 | 700 | 700 |

### Example 6

The following detergent formulations, in accord with the present invention were prepared:

| | P | Q | R | S |
|---|---|---|---|---|
| LAS | 12.0 | 12.0 | 12.0 | 10.0 |
| QAS | 0.7 | 1.0 | - | 0.7 |
| TFAA | - | 1.0 | - | - |
| C25E5/C45E7 | - | 2.0 | - | 0.5 |
| C45E3S | - | 2.5 | - | - |
| CEQ | 2.0 | 1.5 | 1.0 | 1.0 |
| STPP | 30.0 | 18.0 | 15.0 | - |
| Silicate | 9.0 | 7.0 | 10.0 | - |
| Carbonate | 15.0 | 10.5 | 15.0 | 25.0 |
| Bicarbonate | - | 10.5 | - | - |
| DTPMP | 0.7 | 1.0 | - | - |
| SRP 1 | 0.3 | 0.2 | - | 0.1 |
| MA/AA | 2.0 | 1.5 | 2.0 | 1.0 |
| CMC | 0.8 | 0.4 | 0.4 | 0.2 |
| Protease | 0.8 | 1.0 | 0.5 | 0.5 |
| Amylase | 0.8 | 0.4 | - | 0.25 |
| Lipase | 0.2 | 0.1 | 0.2 | 0.1 |
| Cellulase | 0.15 | 0.05 | - | - |
| Photoactivated bleach (ppm) | 70ppm | 45ppm | - | 10ppm |
| Brightener 1 | 0.2 | 0.2 | 0.08 | 0.2 |
| PB1 | 6.0 | 2.0 | - | - |
| NOBS | 2.0 | 1.0 | - | - |
| Balance (Moisture and Miscellaneous) | 100 | 100 | 100 | 100 |

### Example 7

The following detergent formulations, in accord with the present invention were prepared:

| | | T | U | V |
|---|---|---|---|---|
| Blown Powder | | | | |
| | Zeolite A | 10.0 | 15.0 | 6.0 |
| | Sodium sulfate | 19.0 | 5.0 | 7.0 |
| | MA/AA | 3.0 | 3.0 | 6.0 |
| | LAS | 10.0 | 8.0 | 10.0 |
| | C45AS | 4.0 | 5.0 | 7.0 |
| | CEQ | 2.0 | 2.0 | 2.0 |
| | Silicate | - | 1.0 | 7.0 |
| | Soap | - | - | 2.0 |
| | Brightener 1 | 0.2 | 0.2 | 0.2 |
| | Carbonate | 28.0 | 26.0 | 20.0 |
| | DTPMP | - | 0.4 | 0.4 |
| Spray On | | | | |
| | C45E7 | 1.0 | 1.0 | 1.0 |
| Dry additives | | | | |
| | PVPVI/PVNO | 0.5 | 0.5 | 0.5 |
| | Protease | 1.0 | 1.0 | 1.0 |
| | Lipase | 0.4 | 0.4 | 0.4 |
| | Amylase | 0.1 | 0.1 | 0.1 |
| | Cellulase | 0.1 | 0.1 | 0.1 |
| | NOBS | - | 6.1 | 4.5 |
| | PB1 | 1.0 | 5.0 | 6.0 |
| | Sodium sulfate | - | 6.0 | - |
| | Balance (Moisture and Miscellaneous) | 100 | 100 | 100 |

### Example 8

The following high density and bleach-containing detergent formulations, in accord with the present invention were prepared:

| | | W | X | Y |
|---|---|---|---|---|
| Blown Powder | | | | |
| | Zeolite A | 15.0 | 15.0 | 15.0 |
| | Sodim sulfate | 0.0 | 5.0 | 0.0 |
| | LAS | 3.0 | 2.0 | 3.0 |
| | QAS | - | 1.5 | 1.5 |
| | CEQ | 2.0 | 1.5 | 2.0 |
| | DTPMP | 0.4 | 0.4 | 0.4 |
| | CMC | 0.4 | 0.4 | 0.4 |
| | MA/AA | 4.0 | 2.0 | 2.0 |
| Agglomerates | | | | |
| | LAS | 4.0 | 4.0 | 4.0 |
| | TAS | 2.0 | 2.0 | 1.0 |
| | Silicate | 3.0 | 3.0 | 4.0 |
| | Zeolite A | 8.0 | 8.0 | 8.0 |
| | Carbonate | 8.0 | 8.0 | 6.0 |
| Spray On | | | | |
| | Perfume | 0.3 | 0.3 | 0.3 |
| | C45E7 | 2.0 | 2.0 | 2.0 |
| | C25E3 | 2.0 | - | - |
| Dry additives | | | | |
| | Citrate | 5.0 | - | 2.0 |
| | Bicarbonate | - | 3.0 | - |
| | Carbonate | 8.0 | 15.0 | 10.0 |
| | TAED | 6.0 | 2.0 | 5.0 |
| | PB1 | 14.0 | 7.0 | 10.0 |
| | Polyethylene oxide of MW 5,000,000 | - | - | 0.2 |
| | Bentonite clay | - | - | 10.0 |
| | Protease | 1.0 | 1.0 | 1.0 |
| | Lipase | 0.4 | 0.4 | 0.4 |
| | Amylase | 0.6 | 0.6 | 0.6 |
| | Cellulase | 0.6 | 0.6 | 0.6 |
| | Silicone antifoam | 5.0 | 5.0 | 5.0 |
| Dry additives | | | | |
| | Sodium sulfate | 0.0 | 3.0 | 0.0 |
| | Balance (Moisture and Miscellaneous) | 100.0 | 100.0 | 100.0 |
| Density (g/litre) | | 850 | 850 | 850 |

### Example 9

The following high density detergent formulations, in accord with the present invention were prepared:

| | | Z | AA |
|---|---|---|---|
| Agglomerate | | | |
| | C45AS | 11.0 | 14.0 |
| | CEQ | 3.0 | 3.5 |
| | Zeolite A | 15.0 | 6.0 |
| | Carbonate | 4.0 | 8.0 |
| | MA/AA | 4.0 | 2.0 |
| | CMC | 0.5 | 0.5 |
| | DTPMP | 0.4 | 0.4 |
| Spray On | | | |
| | C25E5 | 5.0 | 5.0 |
| | Perfume | 0.5 | 0.5 |
| Dry Adds | | | |
| | HEDP | 0.5 | 0.3 |
| | SKS 6 | 13.0 | 10.0 |
| | Citrate | 3.0 | 1.0 |
| | TAED | 5.0 | 7.0 |
| | Percarbonate | 20.0 | 20.0 |
| | SRP 1 | 0.3 | 0.3 |
| | Protease | 1.4 | 1.4 |
| | Lipase | 0.4 | 0.4 |
| | Cellulase | 0.6 | 0.6 |
| | Amylase | 0.6 | 0.6 |
| | Silicone antifoam | 5.0 | 5.0 |
| | Brightener 1 | 0.2 | 0.2 |
| | Brightener 2 | 0.2 | - |
| | Balance (Moisture and Miscellaneous) | 100 | 100 |
| Density (g/litre) | | 850 | 850 |

## Claims

1. A cationic ester surfactant having the formula: 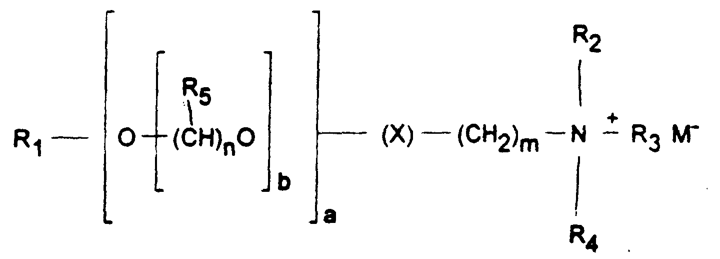 wherein R₁ is a C₅-C₃₁ linear or branched alkyl, alkenyl or alkaryl chain; X is selected from the group consisting of COO, OCO, OCOO, OCONH and NHCOO; R₂, R₃, and R₄ are independently selected from CH₃ and CH₂CH₂OH; and R₅ is independently H or a C₁-C₃ alkyl group; wherein the value of n lies in the range of from 0 to 8, the value of b lies in the range from 0 to 20, the value of a is either 0 or 1, and the value of m is from 3 to 8, m being a halide, methyl sulfate, sulfate, or nitrate.

2. A cationic ester surfactant having the formula: 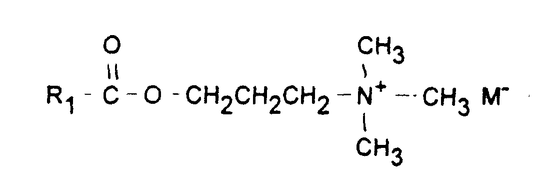 wherein R₁ is a C₁₁-C₁₉ linear or branched alkyl chain.

3. A process for making a cationic ester surfactant according to Claim 2 comprising the steps of:
(i) esterification of a fatty acid (R₁COOH), fatty acid ester (R₁COORₙ) or fatty acid halide (R₁COOHal) with dimethylaminopropanol, in the presence of an acid catalyst; and
(ii) quaternization of the resulting esterification product with a quaternizing agent selected from the group consisting of methyl halide, methyltosylate, dimethylsulfate and any mixtures thereof

4. A process for making a cationic ester surfactant according to Claim 2 comprising the steps of:
(i) esterification of a fatty acid (R₁COOH), fatty acid ester (R₁COORₙ) or fatty acid halide (R₁COOHal) with with 2-halopropanol, in the presence of an acid catalyst; and
(ii) quaternization of the resulting esterification product with trimethylamine.

5. A process according to either of Claims 3 or 4 wherein said quaternization step is conducted in the presence of a solvent selected from the group consisting of ethanol. propylene glycol and C₁₀-C₁₈ fatty alcohol ethoxylate having a degree of ethoxylation of from 3 to 50 ethoxy groups per mole.

6. A surfactant system comprising in combination a cationic ester surfactant according to any of Claims 1 or 2 and an anionic and/or nonionic surfactant.

7. A surfactant system according to Claim 6 comprising both anionic and nonionic surfactant wherein the weight ratio of anionic surfactant to cationic ester surfactant in the surfactant system is from 3:1 to 50:1, and the weight ratio of nonionic surfactant to cationic ester surfactant in the surfactant system is from 3:1 to 50:1.

8. A surfactant system according to either of Claims 6 or 7 wherein said anionic surfactant is an anionic sulfate surfactant.

9. A detergent composition containing a surfactant system according to any of Claims 6 to 8 and one or more additional detergent components selected from the group consisting of an alkalinity system, bleaches, builders, organic polymeric compounds, enzymes, suds suppressors, lime soap dispersants, soil suspension and anti-redeposition agents, corrosion inhibitors and any mixtures thereof.

10. A detergent composition according to Claim 9 comprising
(a) from 1% to 90% by weight of the composition of a surfactant system comprising an anionic surfactant and a cationic ester surfactant according to any of Claims 1 or 2 present at a weight ratio of anionic to cationic ester surfactant of from 3:1 to 15:1; and
(b) from 1.5% to 95% by weight of the composition of an alkalinity system comprising alkaline salts selected from the group consisting of alkali metal or alkaline earth carbonate, bicarbonate, hydroxide or silicate, including crystalline layered silicate, salts and any mixtures thereof.

11. A method of washing laundry in a domestic washing machine in which a dispensing device containing an effective amount of a solid detergent composition according to either of Claims 9 or 10 is introduced into the drum of the washing machine before the commencement of the wash, wherein said dispensing device permits progressive release of said detergent composition into the wash liquor during the wash.

## Patentansprüche

1. Kationisches Ester-Tensid der Formel: 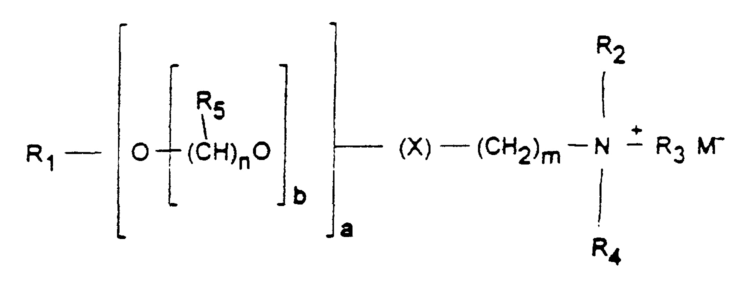 worin R₁ eine lineare oder verzweigte C₅-C₃₁-Alkyl-, Alkenyl- oder Alkarylkette ist; X aus der Gruppe gewählt ist, bestehend aus COO, OCO, OCOO, OCONH und NHCOO; R₂, R₃ und R₄ unabhängig voneinander gewählt sind aus CH₃ und CH₂CH₂OH; und R₅ unabhängig H oder eine C₁-C₃-Alkylgruppe ist; wobei der Wert von n im Bereich von 0 bis 8 liegt, der Wert von b im Bereich von 0 bis 20 liegt, der Wert von a entweder 0 oder 1 ist, und der Wert von m 3 bis 8 beträgt, und M ein Halogenid, Methylsulfat, Sulfat oder Mischung hiervon ist.

2. Kationisches Ester-Tensid der Formel 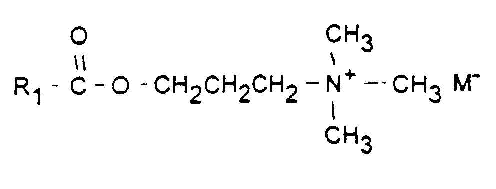 worin R₁ eine lineare oder verzweigte C₁₁-C₁₉-Alkylkette ist.

3. Verfahren zur Herstellung eines kationischen Ester-Tensids nach Anspruch 2, umfassend die Schritte:
(i) Verestern einer Fettsäure (R₁COOH), Fettsäuresters (R₁COORₙ) oder Fettsäurehalogenids (R₁COOHal) mit Dimethylaminopropanol in Gegenwart eines Säurekatalysators; und
(ii) Quaternisieren des resultierenden Veresterungsprodukts mit einem Quaternisierungsmittel, gewählt aus der Methylhalogenid, Methyltosylat, Dimethylsulfat und beliebige Mischungen hiervon umfassenden Gruppe.

4. Verfahren zur Herstellung eines kationischen Ester-Tensids nach Anspruch 2, umfassend die Schritte:
(i) Verestern einer Fettsäure (R₁COOH), Fettsäuresters (R₁COORₙ) oder Fettsäurehalogenids (R₁COOHal) mit 2-Halogenpropanol in Gegenwart eines Säurekatalysators; und
(ii) Quaternisieren des resultierenden Veresterungsprodukts mit Trimethylamin.

5. Verfahren nach mindestens einem der Ansprüche 3 oder 4, wobei der Quaternisierungsschritt in Gegenwart eines Lösungsmittels durchgeführt wird, gewählt aus der Ethanol, Propylenglykol und C₁₀-C₁₈-Fettalkoholethoxylat mit einem Ethoxylierungsgrad von 3 bis 50 Ethoxygruppen pro Mol umfassenden Gruppe.

6. Tensidsystem, umfassend in Kombination ein kationisches Ester-Tensid nach mindestens einem der Ansprüche 1 oder 2 und ein anioniches und/oder nichtionisches Tensid.

7. Tensidsystem nach Anspruch 6, umfassend sowohl anionisches als auch nichtionisches Tensid, wobei das Gewichtsverhältnis von anionischem Tensid zu kationischem Ester-Tensid in dem Tensidsystem 3:1 bis 50:1 beträgt, und das Gewichtsverhältnis von nichtionischem Tensid zu kationischem Ester-Tensid in dem Tensidsystem 3:1 bis 50:1 beträgt.

8. Tensidsystem nach mindestens einem der Ansprüche 6 oder 7, wobei das anionische Tensid ein anionisches Sulfat-Tensid ist.

9. Reinigungsmittelzusammensetzung, enthaltend ein Tensidsystem nach mindestens einem der Ansprüche 6 bis 8 und eine oder mehrere zusätzlichen Reinigungsmittelkomponenten, gewählt aus der Gruppe, bestehend aus einem Alkalinitätssystem, Bleichmitteln, Buildern, organischen polymeren Verbindungen, Enzymen, Schaumunterdrückern, Kalkseifen-Dispergiermitteln, Schmutzsuspendier- und Nichwiederablagerungsmitteln, Korrosionsinhibitoren und beliebige Mischungen hiervon.

10. Reinigungsmittelzusammensetzung nach Anspruch 9, umfassend
(a) 1 bis 90 Gew.-% der Zusammensetzung eines Tensidsystems, umfassend ein anionisches Tensid und ein kationisches Ester-Tensid nach mindestens einem der Ansprüche 1 oder 2, die in einem Gewichtsverhältnis von anionischem zu kationischem Ester-Tensid von 3:1 bis 15:1 vorliegen; und
(b) 1,5 bis 95 Gew.-% der Zusammensetzung eines Alkalinitätssystems, umfassend Alkalisalze, gewählt aus der Alkalimetall- oder Erdalkalimetallcarbonat, -bicarbonat, -hydroxid oder -silicat, einschließlich kristallines Schichtsilicat, Salze und beliebige Mischungen hiervon umfassenden Gruppe.

11. Verfahren zum Waschen von Wäsche in Haushaltswaschmaschinen, in welche eine Dispensiervorrichtung, enthaltend eine wirksame Menge einer festen Waschmittelzusammensetzung nach mindestens einem der Ansprüche 9 oder 10 in die Trommel der Waschmaschine eingebracht wird, bevor die Wäsche beginnt, wobei die Dispensiervorrichtung eine progressive Freisetzung der Waschmittelzusammensetzung in die Waschlauge während der Wäsche ermöglicht.

## Revendications

1. Tensioactif ester cationique de formule: 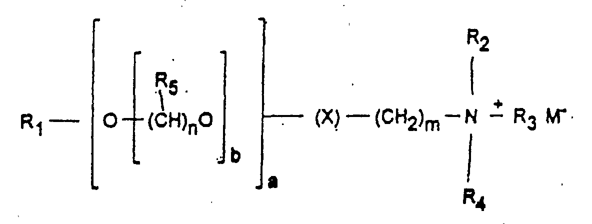 dans laquelle R₁ est une chaîne alkyle, alcényle ou alkaryle linéaire ou ramifiée en C₅-C₃₁; X est choisi dans le groupe constitué par COO, OCO, OCOO, OCONH et NHCOO; R₂, R₃ et R₄ sont choisis indépendamment parmi CH₃ et CH₂CH₂OH; et R5 est indépendamment H ou un groupe alkyle en C₁-C₃; où la valeur de n se situe dans la gamme de 0 à 8, la valeur de b se situe dans la gamme de 0 à 20, la valeur de a est de 0 ou 1 et la valeur de m est de 3 à 8, M étant un halogénure, un méthylsulfate, un sulfate ou un nitrate.

2. Tensioactif ester cationique de formule: 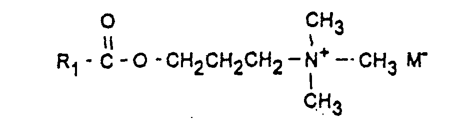 dans laquelle R₁ est une chaîne alkyle linéaire ou ramifiée en C₁₁-C₁₉.

3. Procédé de fabrication d'un tensioactif ester cationique selon la revendication 2, comprenant les étapes consistant à:
(i) estérifier un acide gras (R₁COOH), un ester d'acide gras (R₁COORₙ) ou un halogénure d'acide gras (R₁COOHal) avec du diméthylaminopropanol, en présence d'un catalyseur acide; et
(ii) quaterniser le produit d'estérification résultant avec un agent quaternisant choisi dans le groupe constitué par un halogénure de méthyle, un tosylate de méthyle, un sulfate de diméthyle et leurs mélanges.

4. Procédé de fabrication d'un tensioactif ester cationique selon la revendication 2, comprenant les étapes consistant à:
(i) estérifier un acide gras (R₁COOH), un ester d'acide gras (R₁COORₙ) ou un halogénure d'acide gras (R₁COOHal) avec un 2-halogénopropanol, en présence d'un catalyseur acide; et
(ii) quaterniser le produit d'estérification résultant avec de la triméthylamine.

5. Procédé selon l'une quelconque des revendications 3 ou 4, dans lequel ladite étape de quaternisation est réalisée en présence d'un solvant choisi dans le groupe constitué par l'éthanol, le propylèneglycol et un éthoxylate d'alcool gras en C₁₀-C₁₈ ayant un degré d'éthoxylation de 3 à 50 groupes éthoxy par mole.

6. Système tensioactif comprenant une combinaison d'un tensioactif ester cationique selon l'une quelconque des revendications 1 ou 2 et d'un tensioactif anionique et/ou non ionique.

7. Système tensioactif selon la revendication 6, comprenant à la fois un tensioactif anionique et un tensioactif non ionique, dans lequel le rapport pondéral du tensioactif anionique au tensioactif ester cationique dans le système tensioactif est de 3/1 à 50/1, et le rapport pondéral du tensioactif non ionique au tensioactif ester cationique dans le système tensioactif est de 3/1 à 50/1.

8. Système tensioactif selon l'une quelconque des revendications 6 ou 7, dans lequel ledit tensioactif anionique est un tensioactif sulfate anionique.

9. Composition détergente contenant un système tensioactif selon l'une quelconque des revendications 6 à 8 et un ou plusieurs constituants détergents supplémentaires choisis dans le groupe constitué par un système d'alcalinité, des agents de blanchiment, des adjuvants, des composés polymères organiques, des enzymes, des antimousses, des dispersants de savon de calcaire, des agents de mise en suspension et antiredéposition des salissures, des inhibiteurs de corrosion, et des mélanges quelconques de ceux-ci.

10. Composition détergente selon la revendication 9, comprenant
(a) de 1% à 90%, en poids de la composition, d'un système tensioactif comprenant un tensioactif anionique et un tensioactif ester cationique selon l'une quelconque des revendications 1 ou 2, présents dans un rapport pondéral du tensioactif anionique au tensioactif ester cationique de 3/1 à 15/1, et
(b) de 1,5% à 95%, en poids de la composition, d'un système d'alcalinité comprenant des sels alcalins choisis dans le groupe constitué par le carbonate, bicarbonate, hydroxyde ou silicate de métal alcalin ou de métal alcalino-terreux, notamment les phyllosilicates cristallins, les sels et mélanges quelconques de ceux-ci.

11. Procédé de lavage du linge dans une machine à laver domestique dans lequel un dispositif distributeur contenant une quantité efficace d'une composition détergente solide selon l'une quelconque des revendications 9 ou 10 est introduit dans le tambour de la machine à laver avant le début du lavage, dans lequel ledit dispositif distributeur permet une libération progressive de ladite composition détergente dans la liqueur de lavage au cours du lavage.
